# EUROPEAN PATENT SPECIFICATION

(11) **EP 3 755 429 B1**
(45) Date of publication and mention of the grant of the patent: **31.05.2023**
(21) Application number: 19722364.7
(22) Date of filing: 17.04.2019
(51) Int. Cl.: A61N 1/372, A61N 1/36

(54) **SYSTEM TO OPTIMIZE ANODIC/CATHODIC STIMULATION MODES**
SYSTEM ZUR OPTIMIERUNG ANODISCHER/KATHODISCHER STIMULATIONSMODI
SYSTÈME D'OPTIMISATION DE MODES DE STIMULATION ANODIQUE/CATHODIQUE

(30) Priority: 27.04.2018 US 201862663905 P
(43) Date of publication of application: 30.12.2020
(73) Proprietor: Boston Scientific Neuromodulation Corporation, Valencia, CA 91355 (US)
(72) Inventor: KOTHANDARAMAN, Sridhar, Valencia, CA 91354 (US); MUSTAKOS, Richard, Simi Valley, CA 93063 (US); MOFFITT, Michael A., Saugus, CA 91390 (US); SHAH, Chirag, Valencia, CA 91354 (US); YOO, Peter J., Burbank, CA 91504 (US); GUNNA SRINIVASAN, Vikrant Venkateshwar, Los Angeles, CA 90034 (US)
(74) Representative: Vossius & Partner Patentanwälte Rechtsanwälte mbB
(86) International application number: PCT/US2019/027935
(87) International publication number: WO 2019/209594

(56) References cited:
- US-A1- 2013 116 751
- US-A1- 2014 277 267
- US-A1- 2016 220 820
- US-A1- 2018 071 515

## Description

### FIELD OF THE TECHNOLOGY

The present disclosure relates to techniques to optimize stimulation when both anodic and cathodic modes of stimulation are employed.

### INTRODUCTION

Implantable stimulation devices are devices that generate and deliver electrical stimuli to nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will focus primarily on the use of the disclosed techniques within a Deep Brain Stimulation (DBS) system, such as is disclosed in U.S. Patent Application Publication No. 2013/0184794. However, the disclosed techniques may find applicability in the context of any implantable medical device or implantable medical device system. US2013116751 A1 discloses a neurostimulation system which has a controller that instructs a neurostimulator to convey electrical current to the electrodes based on new electrical current distribution.

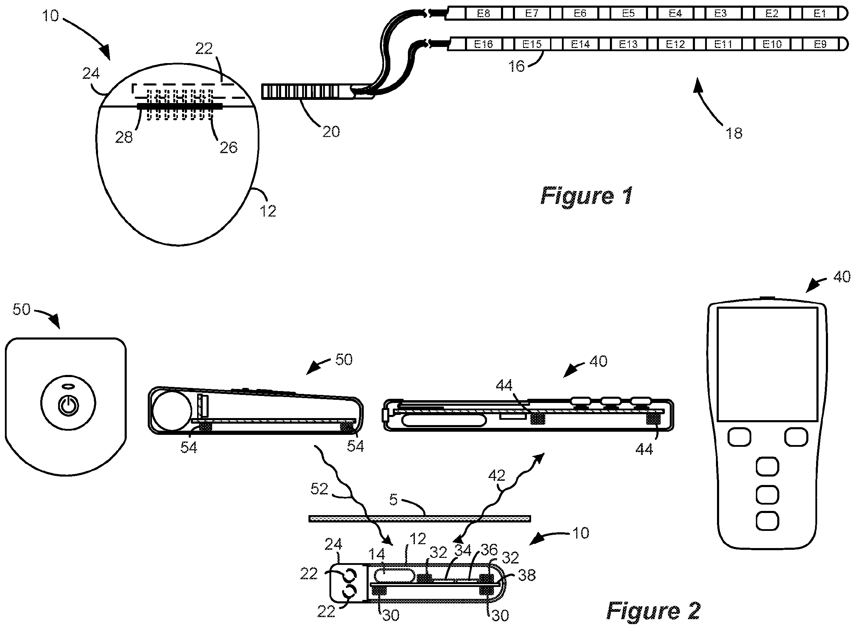

As shown in Figure 1, a DBS system typically includes an implantable pulse generator (IPG) 10 (or an implantable medical device, more generally), which includes a biocompatible device case 12 that is formed from a metallic material such as titanium. The case 12 typically comprises two components that are welded together, and it holds the circuitry and battery 14 (Fig. 2) necessary for the IPG 10 to function. The battery 14 may be either rechargeable or primary (non-rechargeable) in nature. The IPG 10 is coupled to electrodes 16 via one or more electrode leads 18 (two of which are shown). The proximal ends of the leads 18 include electrode terminals 20 that are coupled to the IPG 10 at one or more connector blocks 22 fixed in a header 24, which can comprise an epoxy for example. Contacts in the connector blocks 22 make electrical contact with the electrode terminals 20, and communicate with the circuitry inside the case 12 via feedthrough pins 26 passing through a hermetic feedthrough 28 to allow such circuitry to provide stimulation to or monitor the various electrodes 16. The feedthrough assembly 28, which is typically a glass, ceramic, or metallic material, is affixed to the case 12 at its edges to form a hermetic seal. In the illustrated system, there are sixteen electrodes 16 split between two leads 18, although the number of leads and electrodes is application specific and therefore can vary.

As shown in Figure 2, IPG 10 contains a charging coil 30 for wireless charging of the IPG's battery 14 using an external charging device 50, assuming that battery 14 is a rechargeable battery. If IPG 10 has a primary battery 14, charging coil 30 in the IPG 10 and external charger 50 can be eliminated. IPG 10 also contains a telemetry coil antenna 32 for wirelessly communicating data with an external controller device 40, which is explained further below. In other examples, antenna 32 can comprise a short-range RF antenna such as a slot, patch, or wire antenna. IPG 10 also contains control circuitry such as a microcontroller 34, and one or more Application Specific Integrated Circuit (ASICs) 36, which can be as described for example in USP 8,768,453. ASIC(s) 36 can include current generation circuitry for providing stimulation pulses at one or more of the electrodes 16 and may also include telemetry modulation and demodulation circuitry for enabling bidirectional wireless communications at antenna 32, battery charging and protection circuitry couplable to charging coil 30, DC-blocking capacitors in each of the current paths proceeding to the electrodes 16, etc. Components within the case 12 are integrated via a printed circuit board (PCB) 38.

Figure 2 further shows the external components referenced above, which may be used to communicate with the IPG 10, in plan and cross section views. External controller 40 may be used to control and monitor the IPG 10 via a bidirectional wireless communication link 42 passing through a patient's tissue 5. For example, the external controller 40 may be used to provide or adjust a stimulation program for the IPG 10 to execute that provides stimulation to the patient. The stimulation program may specify a number of stimulation parameters, such as which electrodes are selected for stimulation; whether such active electrodes are to act as anodes or cathodes; and the amplitude (e.g., current), frequency, and duration of stimulation at the active electrodes, assuming such stimulation comprises stimulation pulses as is typical.

Communication on link 42 can occur via magnetic inductive coupling between a coil antenna 44 in the external controller 40 and the IPG 10's telemetry coil 32 as is well known. Typically, the magnetic field comprising link 42 is modulated via Frequency Shift Keying (FSK) or the like, to encode transmitted data. For example, data telemetry via FSK can occur around a center frequency of fc = 125 kHz, with a 129 kHz signal representing transmission of a logic '1' bit and 121 kHz representing a logic '0' bit. However, transcutaneous communications on link 42 need not be by magnetic induction, and may comprise short-range RF telemetry (e.g., Bluetooth, WiFi, Zigbee, MICS, etc.) if antennas 44 and 32 and their associated communication circuitry are so configured. The external controller 40 is generally similar to a cell phone and includes a hand-holdable, portable housing.

External charger 50 provides power to recharge the IPG 10's battery 14 should that battery be rechargeable. Such power transfer occurs by energizing a charging coil 54 in the external charger 50, which produces a magnetic field comprising transcutaneous link 52, which may occur with a different frequency (f2 = 80 kHz) than data communications on link 42. This magnetic field 52 energizes the charging coil 30 in the IPG 10, which is rectified, filtered, and used to recharge the battery 14. Link 52, like link 42, can be bidirectional to allow the IPG 10 to report status information back to the external charger 50, such as by using Load Shift Keying as is well-known. For example, once circuitry in the IPG 10 detects that the battery 14 is fully charged, it can cause charging coil 30 to signal that fact back to the external charger 50 so that charging can cease. Like the external controller 40, external charger 50 generally comprises a hand-holdable and portable housing.

### SUMMARY

A system is disclosed having an implantable medical device that is connectable to an electrode lead having a plurality of electrodes; and a non-transitory computer-readable medium having instructions that are executable by control circuitry to cause the control circuitry to generate a graphical user interface that is configured to receive one or more inputs to specify one or more parameters of a transition between a first stimulation mode and a second stimulation mode, wherein the first stimulation mode defines stimulation of a first polarity to be issued at a set of the electrodes and the second stimulation mode defines stimulation of a second polarity to be issued at the set of the electrodes, wherein the first polarity is opposite of the second polarity; and communicate stimulation parameters based on the one or more parameters of the transition to the implantable medical device.

The one or more inputs may include an input that specifies a duration of the transition. The one or more inputs may include an input that specifies a type of the transition, which type may be selectable as either a linear transition type or a user-customizable transition type. The linear transition type may specify a linear increase of an adjustment variable of the first stimulation mode and a linear decrease of the adjustment variable of the second stimulation mode over a transition period. The one or more inputs may include an input that specifies the adjustment variable, and the adjustment variable may be selectable as either pulse width or pulse amplitude.

The user-customizable transition type may specify a user-customizable adjustment of an adjustment variable of the first stimulation mode and the second stimulation mode over a transition period. The graphical user interface may be configured to receive one or more sets of values that specify a proportion of a configured value of the adjustment variable over the transition period. A first one of the sets of values may specify the proportion as increasing from zero to unity and a second one of the sets of values may specify the proportion as decreasing from unity to zero. A first one of the sets of values may specify the proportion of the configured value of the adjustment variable for the first stimulation mode and a second one of the sets of values may specify the proportion of the configured value of the adjustment variable for the second stimulation mode. A first one of the sets of values may specify the proportion of the configured value of the adjustment variable for a currently-active one of the first stimulation mode and the second stimulation mode and a second one of the sets of values may specify the proportion of the configured value of the adjustment variable for a currently-inactive one of the first stimulation mode and the second stimulation mode. The one or more inputs may include an input that specifies the adjustment variable, and the adjustment variable may be selectable as either pulse width or pulse amplitude.

The one or more inputs may include an input that specifies a time apportionment of the first stimulation mode and the second stimulation mode during a transition period. The graphical user interface may include an indication of a relative energy usage of the first stimulation mode and the second stimulation mode. The graphical user interface may provide a visualization of an impact on energy usage by the implantable medical device for proposed modifications to the first stimulation mode and the second stimulation mode. The proposed modifications may include an adjustment of a first time apportionment of the first stimulation mode and the second stimulation mode. The proposed modifications may include an adjustment of a second time apportionment during which the first time apportionment is overridden. The visualization of the impact on energy usage may include an indication of an estimated recharge interval based on the proposed modifications. The visualization of the impact on energy usage may include an indication of an estimated battery life based on the proposed modifications.

A system is disclosed comprising an implantable medical device that is connectable to an electrode lead having a plurality of electrodes; and a non-transitory computer-readable medium having instructions that are executable by control circuitry to cause the control circuitry to generate a graphical user interface that is configured to receive a first set of inputs that specify adjustments to a parameter of a first stimulation mode over a time period and a second set of inputs that specify adjustments to a parameter of a second stimulation mode over the time period, wherein the first stimulation mode defines stimulation of a first polarity to be issued at a set of the electrodes and the second stimulation mode defines stimulation of a second polarity to be issued at the set of the electrodes, wherein the first polarity is opposite of the second polarity; and communicate stimulation parameters that are based on the first set of inputs and the second set of inputs to the implantable medical device.

The graphical user interface may be further configured to receive an input that specifies a duration of the time period. The graphical user interface may be further configured to receive an input that specifies the parameter of the first stimulation mode and the parameter of the second stimulation mode. The parameter of the first stimulation mode and the parameter of the second stimulation mode may be selectable as either pulse width or pulse amplitude.

The first set of inputs may specify a proportion of a configured value of the parameter of the first stimulation mode at first points in the time period and the second set of inputs may specify a proportion of a configured value of the parameter of the second stimulation mode at second points in the time period. The graphical user interface may be further configured to receive an input that specifies a fitting technique that represents the first set of inputs as a first function of time and the second set of inputs as a second function of time. The graphical user interface may be further configured to receive an input that specifies a time apportionment of the first stimulation mode and the second stimulation mode. The stimulation parameters may define stimulation based on the time apportionment and a repeating application of the first and second functions of time.

The graphical user interface may further comprise an indication of a relative energy usage of the first stimulation mode and the second stimulation mode. The graphical user interface may provide a visualization of an impact on energy usage by the implantable medical device for proposed modifications to the first stimulation mode and the second stimulation mode. The proposed modifications may include an adjustment of a first time apportionment of the first stimulation mode and the second stimulation mode. The proposed modifications may include an adjustment of a second time apportionment during which the first time apportionment is overridden. The visualization of the impact on energy usage may include an indication of an estimated recharge interval based on the proposed modifications. The visualization of the impact on energy usage may include an indication of an estimated battery life based on the proposed modifications. The relative energy usage of the first stimulation mode and the second stimulation mode may be calculated based on tissue impedance during the first stimulation mode and the second stimulation mode. A plurality of measurements of the tissue impedance may be obtained during stimulation in each of the first and second stimulation modes and the tissue impedance for each of the first and second stimulation modes may be based on an average of the plurality of measurements for that particular stimulation mode. The graphical user interface may further include controls that enable a user to evaluate an effect of settings for the first stimulation mode and the second stimulation mode on programming limits.

The graphical user interface may be configured to default to presenting a first interface for configuring the first stimulation mode when the implantable medical device comprises a first type of battery and to default to presenting a second interface for configuring the second stimulation mode when the implantable medical device comprises a second type of battery. The first type of battery may be a rechargeable battery and the second type of battery may be a primary cell battery.

A system is disclosed comprising an implantable medical device that is connectable to an electrode lead having a plurality of electrodes; and a non-transitory computer-readable medium having instructions that are executable by control circuitry to cause the control circuitry to receive a first input that corresponds to a first stimulation location and a second input that corresponds to a second stimulation location; define stimulation regimes corresponding to each of the first stimulation location and the second stimulation location for each of a first stimulation mode and a second stimulation mode, wherein the first stimulation mode defines stimulation of a first polarity and the second stimulation mode defines stimulation of a second polarity that is opposite of the first polarity; define a path that connects the stimulation regimes; receive a selection of a position along the path; and communicate stimulation parameters that are based on the selected position to the implantable medical device.

A system is disclosed comprising an implantable medical device that is connectable to an electrode lead having a plurality of electrodes; and a non-transitory computer-readable medium having instructions that are executable by control circuitry to cause the control circuitry to receive one or more inputs that specify a time apportionment of a first stimulation mode and a second stimulation mode, wherein the first stimulation mode defines pulses of a first polarity to be issued at a set of the electrodes and the second stimulation mode defines pulses of a second polarity to be issued at the set of the electrodes, wherein the first polarity is opposite of the second polarity; and communicate stimulation parameters based on the one or more inputs to the implantable medical device.

### BRIEF DESCRIPTION OF THE DRAWINGS

Figure 1 shows an implantable pulse generator (IPG) with an electrode array.
Figure 2 shows a cross section of the IPG of Figure 1 as implanted in a patient, as well as external devices that support the IPG, including an external charger and external controller.
Figure 3 shows implantation of the IPG in a patient in a Deep Brain Stimulation (DBS) application.
Figure 4 shows an electrode lead having segmented electrodes as may be used in a DBS application.
Figure 5 shows components of a clinician's programmer system, including components for communicating with a neurostimulator such as an IPG.
Figure 6 shows an example of Digital-to-Analog Converter (DAC) circuitry as arranged to generate a biphasic square wave pulse.
Figure 7 illustrates a programming interface that enables a user to configure both anodic and cathodic stimulation modes in accordance with an aspect of the disclosure.
Figure 8 illustrates various interfaces that enable a user to configure the interaction between configured anodic and cathodic stimulation modes in accordance with an aspect of the disclosure.
Figure 9 illustrates example waveforms that show various pulse-based apportionments of anodic and cathodic stimulation modes in accordance with an aspect of the disclosure.
Figure 10 shows aspects of time period-based apportionments of anodic and cathodic stimulation modes in accordance with an aspect of the disclosure.
Figure 11 illustrates an example waveform corresponding to a portion of a linear transition from an anodic stimulation mode to a cathodic stimulation mode in accordance with an aspect of the disclosure.
Figure 12 illustrates an example waveform corresponding to an example user-customized transition from an anodic stimulation mode to a cathodic stimulation mode in accordance with an aspect of the disclosure.
Figure 13 illustrates an example waveform corresponding to an example user-customized interleaving of an anodic stimulation mode and a cathodic stimulation mode in accordance with an aspect of the disclosure.
Figure 14 illustrates a programming interface that enables a user to configure an anodic/cathodic weave in accordance with an aspect of the disclosure.
Figure 15 illustrates transitions between selected electrodes for an example anodic/cathodic weave in accordance with an aspect of the disclosure.
Figures 16A-16F illustrate waveforms at selected electrodes for each of the transitions illustrated in Figure 15 in accordance with an aspect of the disclosure.
Figure 17 illustrates a programming interface that enables a user to configure an anodic/cathodic weave in accordance with an aspect of the disclosure.
Figure 18 illustrates an energy management interface in accordance with an aspect of the disclosure.
Figure 19 illustrates an example computing environment on which software that provides various described functionality may be executed in accordance with an aspect of the disclosure.

### DETAILED DESCRIPTION

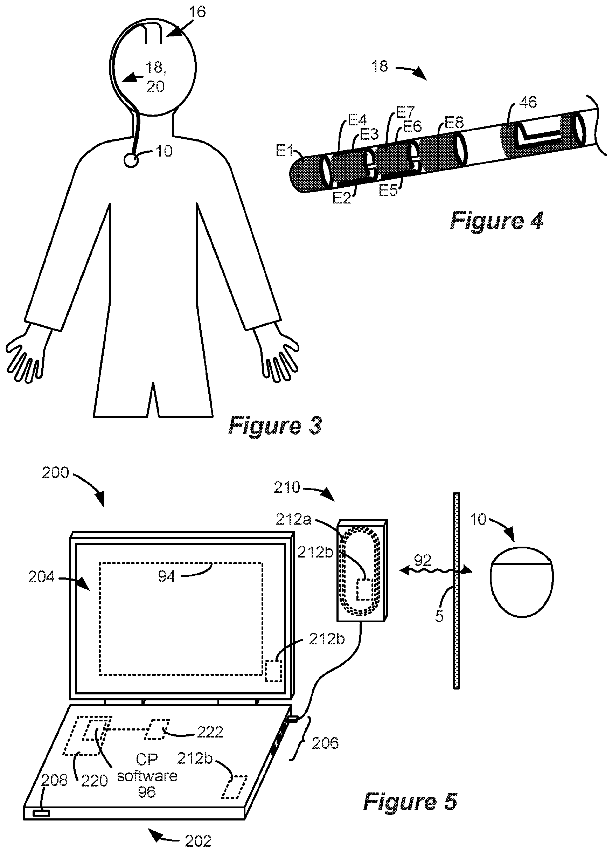

In a DBS application, as is useful in the treatment of neurological disorders such as Parkinson's disease, the IPG 10 is typically implanted under the patient's clavicle (collarbone), and the leads 18 are tunneled through the neck and between the skull and the scalp where the electrodes 16 are implanted through holes drilled in the skull in the left and right sides of the patient's brain, as shown in Figure 3. Specifically, the electrodes 16 may be implanted in the subthalamic nucleus (STN), the pedunculopontine nucleus (PPN), or the globus pallidus internus (GPi). Stimulation therapy provided by the IPG 10 has shown promise in reducing the symptoms of neurological disorders, including rigidity, bradykinesia, tremor, gait and turning impairment, postural instability, freezing, arm swing, balance impairment, and dystonia.

While Figure 1 generically illustrates the electrodes 16 as aligned linearly along a lead 18, electrode leads 18 for DBS applications commonly include segmented electrodes that allow for directional control of stimulation. The electrode lead 18 in Figure 4 includes multiple circumferential (or ring) electrodes and multiple segmented electrodes. In particular, electrodes E1 and E8 are circumferential electrodes that extend around the circumference of the lead 18 while electrodes E2-E7 are segmented electrodes. As used herein, segmented electrodes refer to electrodes that do not extend fully around the perimeter of an electrode lead 18. In the illustrated embodiment, the segmented electrodes are arranged with three electrodes at a particular axial position, each segmented electrode spanning an approximately 90 degree arc around the lead 18 with approximately 30 degree spaces between neighboring segmented electrodes. Although a particular example of a lead is illustrated in Figure 4, the type and placement of electrodes 16 along a lead is application specific and therefore can vary. For example, a lead may include more or fewer segmented electrodes at a given axial position and more or fewer circumferential electrodes in addition to the segmented electrodes. As will be understood, because the segmented electrodes are separated by a non-conductive break, electrical stimulation that is directed to a segmented electrode propagates outward in the direction of the electrode rather than uniformly about the lead 18 as with circumferential electrodes. The lead 18 additionally includes a marker 46 that is aligned with segmented electrodes E2 and E5. The marker 46 provides a visual indication of the lead's orientation prior to implantation as well as a radiological indication of the lead's orientation after implantation.

As mentioned above, the electrical stimulation that the IPG 10 is capable of delivering is highly customizable with respect to selected electrodes, current amplitude and polarity, pulse duration, pulse frequency, etc. Due to uncertainties in the location of electrodes with respect to neural targets, the physiological response of a patient to stimulation patterns, and the nature of the electrical environment within which the electrodes are positioned, the stimulation parameters that might provide effective stimulation therapy for a particular patient are typically determined using a trial and error approach. Thus, after the leads are implanted, an initial programming session is typically performed to customize the parameters of the stimulation provided by the IPG 10 to obtain the greatest benefit for the patient. While not common in DBS applications due to the dangers of having externalized leads or lead extensions, in other applications such as spinal cord stimulation (SCS), it is common for the initial programming session to be performed after lead implantation using an external trial stimulator that mimics the operation of the IPG 10 and that is coupled to the implanted leads 18 but is not itself implanted.

Referring to Figure 5, the initial programming is typically performed by communicating different stimulation programs from a clinician's programmer system (CP System) 200 to the IPG 10 and observing the patient's responses to the IPG 10's execution of the different programs. For a DBS application, a clinician may observe the extent to which the current stimulation program decreases the effects of the patient's neurological disorder (e.g., the extent to which the stimulation program decreases the degree of tremor) as well as any side effects induced as a result of the stimulation program. As shown, CP system 200 can comprise a computing device 202, such as a desktop, laptop, or notebook computer, a tablet, a mobile smart phone, a Personal Data Assistant (PDA)-type mobile computing device, etc. (hereinafter "CP computer"). In Figure 5, CP computer 202 is shown as a laptop computer that includes typical computer user interface means such as a screen 204, a mouse, a keyboard, speakers, a stylus, a printer, etc., not all of which are shown for convenience.

Also shown in Figure 5 is an accessory communication head 210 that is couplable to a port of the CP computer 202, such as a USB port 206, and that is specific to the CP computer 202's operation as a neurostimulator controller. Communication between the CP system 200 and the IPG 10 may comprise magnetic inductive or short-range RF telemetry schemes (as described above with respect to communications between the IPG 10 and the programmer 40), and in this regard the IPG 10 and the CP computer 202 and/or the communication head 210 (which can be placed proximate to the IPG 10) may include antennas compliant with the telemetry means chosen. For example, the communication head 210 can include a coil antenna 212a, a short-range RF antenna 212b, or both. The CP computer 202 may also communicate directly with the IPG 10, for example using an integral short-range RF antenna 212b, without the use of the communication head 210.

If the CP system 200 includes a short-range RF antenna (either in CP computer 202 or communication head 210), such antenna can also be used to establish communication between the CP system 200 and other devices, and ultimately to larger communication networks such as the Internet. The CP system 200 can typically also communicate with such other networks via a wired link provided at an Ethernet or network port 208 on the CP computer 202, or with other devices or networks using other wired connections (e.g., at USB ports 206).

To test different stimulation parameters during the initial programming session, a user interfaces with a clinician programmer graphical user interface (CP GUI) 94 provided on the display 204 of the CP computer 202. As one skilled in the art understands, the CP GUI 94 can be rendered by execution of CP software 96 on the CP computer 202, which software may be stored in the CP computer 202's non-volatile memory 220. One skilled in the art will additionally recognize that execution of the CP software 96 in the CP computer 202 can be facilitated by control circuitry 222 such as a microprocessor, microcomputer, an FPGA, other digital logic structures, etc., which is capable of executing programs in a computing device. Such control circuitry 222 when executing the CP software 96 will in addition to rendering the CP GUI 94 cause the CP computer 202 to communicate the stimulation parameters to the IPG 10 using a suitable antenna 212a or 212b, either in the communication head 210 or the CP computer 202 as explained earlier. The CP software 96 enables a user to select the type of electrode lead(s) that have been implanted (e.g., from a list of leads that are configured in the software 96) and to customize the stimulation parameters using the available electrodes on the implanted lead. In this way, the user can communicate different stimulation parameters to the IPG 10 for execution to observe the effects of the various parameters and to hone in on the appropriate settings for the patient.

The stimulation parameters that are communicated to the IPG 10 are ultimately converted to control signals that are distributed to one or more Digital-to-Analog Converters (DACs) 72 in the IPG 10's stimulation circuitry to form pulses defined by the stimulation parameters at the selected electrodes. Traditionally, DBS stimulation has involved the periodic application of electrical pulses with one or more lead-based electrodes 16 (i.e., one or more electrodes 16 that are carried on the lead 18 and thus implanted in the region of interest such as the brain) acting as the cathode and the case 12 acting as the anode.

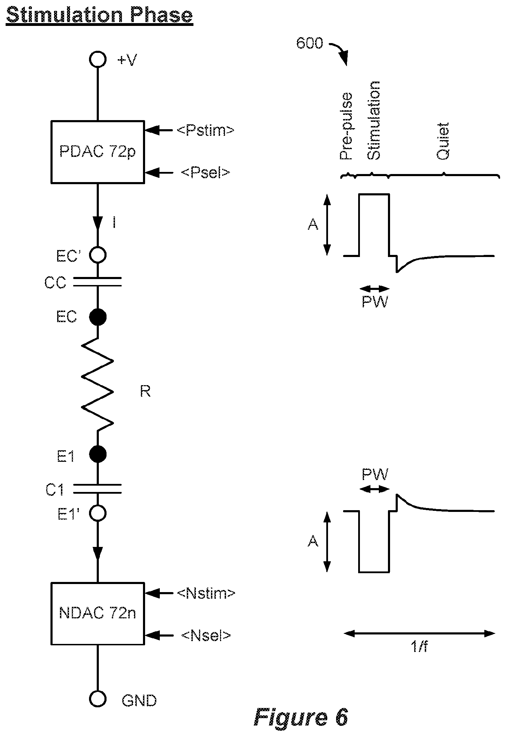

Figure 6 shows a simple example of DAC circuitry 72 as arranged to provide a traditional square wave pulse 600 of this type using electrode E1 as the selected lead-based electrode 16. DAC circuitry 72 as shown comprises two portions, denoted as PDAC 72p and NDAC 72n. These portions of DAC circuitry 72 are so named due to the polarity of the transistors used to build them and the polarity of the current they provide. Thus, PDAC 72p is formed from P-channel transistors and is used to source a current +I to the patient's tissue R via a selected electrode operating as an anode, and NDAC 72n is formed of N-channel transistors and is used to sink current -I from the patient's tissue via a selected electrode operating as a cathode. It is important that current sourced to the tissue at any given time equal that sunk from the tissue to prevent charge from building in the tissue, although more than one lead-based electrode 16 may be operable at a given time.

PDAC 72p and NDAC 72n are current sources that receive digital control signals, denoted <Pstim> and <Nstim> respectively, to generate current of a prescribed amplitude at appropriate times. More specifically, PDAC 72p and NDAC 72n include current-mirrored transistors for mirroring (amplifying) a reference current Iref to produce pulses with a specified amplitude. Although the DAC circuitry 72 (PDAC 72p and NDAC 72n) may be dedicated at each of the electrodes and thus may be activated only when its associated electrode is to be selected as an anode or cathode, see, e.g., USP 6,181,969, the illustrated example assumes that one or more DACs (or one or more current sources within a DAC ) are distributed to a selected electrode by a switch matrix (not shown), and control signals <Psel> and <Nsel> are used to control the switch matrix and establish the connection between the selected electrode and the PDAC 72p or NDAC 72n.

In the example shown, control signals <Pstim>, <Nstim>, <Psel>, and <Nsel> prescribe the various parameters of the square wave pulse 600. The pulse 600 is defined by multiple phases that include a pre-pulse phase, a stimulation phase, and a quiet phase. During the stimulation phase, current I (having amplitude A) is sourced from the PDAC 72p to electrode node EC' (a node in the IPG 10's current generation circuitry that is coupled to the case 12 through a blocking capacitor CC) for a duration PW. From electrode node EC', the current I flows through the blocking capacitor CC to the case 12 (operating as electrode EC). The NDAC 72n pulls the current I through the patient's tissue R from electrode E1 through the blocking capacitor C1 and to the electrode node E1' over the same duration PW. In the monophasic type of stimulation that is illustrated, charge that has built up on the blocking capacitors during the stimulation phase is recovered using passive recovery (illustrated as the decaying charge in the quiet phase) during the quiet phase as is known. Alternatively, a recovery phase pulse of opposite polarity may be applied at the selected electrodes following the stimulation phase pulse to recover charge that has built up on the blocking capacitors during the stimulation phase. However, even when such active recovery is employed, it has been assumed that only the stimulation phase pulse provides therapeutically effective therapy. Although pulses of different types and shapes may be formed, the examples in the remainder of this application depict monophasic square wave pulses (with passive recovery indication omitted). It should be noted though, that the application is relevant to pulses of different types and the polarity of a particular pulse is assumed to describe the polarity during an active stimulation phase. In addition, this application is relevant to coordinated reset therapy, which can include anodic coordinated reset therapy, mixed anodic and cathodic coordinated reset therapy, and cathodic coordinated reset therapy.

The PDAC 72p and NDAC 72n along with the intervening tissue R complete a circuit between a power supply +V and ground. The compliance voltage +V is adjustable to an optimal level to ensure that current pulses of a prescribed amplitude can be produced without unnecessarily wasting IPG power. While a single pulse 600 is illustrated, such a pulse is typically repeated in succession and the duration of the single period of the pulse 600 defines the stimulation frequency f.

Traditional DBS programming has focused on the selection of the one or more lead-based electrodes 16, the pulse width, the pulse amplitude, and the stimulation frequency that provides the most effective therapy for the patient. The polarity of the lead-based electrodes 16 during the stimulation phase, however, has not been a customizable parameter of traditional DBS stimulation as it has been considered that only cathodic stimulation at the particular area of interest (i.e., the tissue within which the leads are implanted) is therapeutically effective. Recently, it has been observed that anodic stimulation (i.e., stimulation in which one or more selected lead-based electrodes 16 operate as the anode) can provide beneficial therapeutic effects. It has further been observed that anodic stimulation operates via a different biological mechanism than traditional cathodic stimulation and that the different types of stimulation provide different therapeutic effects. The inventors recognize that it is desirable to provide a patient (or the patient's clinician) with the ability to configure the IPG 10 to provide anodic or cathodic stimulation or some mixture of the different stimulation types, to transition between anodic and cathodic stimulation modes in user-customizable ways that provide the most effective therapy, and to apportion the amount of time during which anodic and cathodic stimulation are provided by the IPG 10.

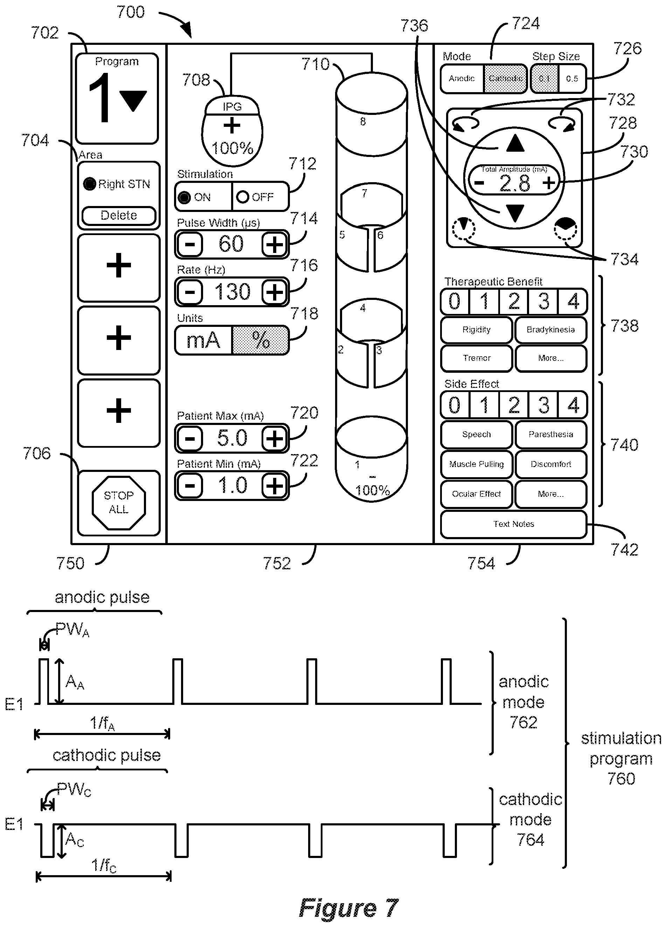

Figure 7 illustrates an example of a programming interface 700, which is provided as part of an improved CP GUI 94' that is generated through execution of improved CP software 96' and that enables the user to configure stimulation parameters that specify both anodic and cathodic stimulation. Each of the interfaces described in the remainder of this application are graphical user interfaces that are part of the improved CP GUI 94'. The programming interface 700 includes a first window 750, a second window 752, and a third window 754. The first window 750 includes a program selector 702, an area selector 704, and a stimulation termination selector 706. The program selector 702 enables the user to select which one of multiple stimulation programs is being configured. In an example embodiment, the programming interface 700 enables up to four stimulation programs to be configured for simultaneous execution, but more or fewer stimulation programs may be supported in accordance with the capabilities of the IPG 10. The area selector 704 enables the user to select which one of multiple areas is being configured for the selected stimulation program. In the illustrated example, the programming interface 700 supports the configuration of up to four areas, each of which corresponds to an implanted electrode lead 18. The areas are configured via another portion of the CP GUI 94' where each area is given a descriptive name and matched with the particular electrode lead 18 that is implanted in the described area. In the illustrated example, a single area has been configured and it has been assigned a descriptor of "Right STN". This notation indicates that the stimulation parameters that are configured for the "Right STN" area correspond to the electrode lead 18 that is implanted in the patient's right subthalamic nucleus. The stimulation termination selector 706 can be utilized to suspend all stimulation that is being delivered by the IPG 10.

When a particular area is selected in the first window 750, a representation of the type of lead 18 that is matched with the selected area is displayed in the second window 752. In the illustrated example, a segmented lead 18 has been implanted in the patient's right subthalamic nucleus and the area "Right STN" has been matched with this type of lead in the configuration of the "Right STN" area. Thus, when the "Right STN" area is selected in the first window 750, a representation 710 of the lead 18 is depicted in the second window 752. The second window 752 additionally includes a representation 708 of the IPG 10, a stimulation on/off selector 712, a pulse width selector 714, a frequency selector 716, a units selector 718, a maximum current selector 720, and a minimum current selector 722. The stimulation on/off selector 712 enables the user to turn stimulation on or off for the selected program. The pulse width selector 714 enables the user to increase or decrease the stimulation and recovery pulse widths for the selected program and the selected stimulation mode (i.e., anodic or cathodic), which stimulation mode is selected via the stimulation mode selector 724 as described below. The frequency selector 716 enables the user to increase or decrease the frequency at which pulses are applied for the selected program and the selected stimulation mode. The units selector 718 enables the user to specify whether the allocation of stimulation current is depicted on the lead representation 710 and IPG representation 708 in terms of the percent of the total stimulation current or the actual amplitude of the current in milliamps. In the illustrated example, the units selector 718 has been selected to identify current allocation in terms of the percentage of total stimulation current and the lead and IPG representations indicate that 100% of the cathodic current is allocated to electrode E1 and 100% of the anodic current is allocated to the IPG case 12. The allocation of current amongst the available electrodes is configured within the third window 754 as is described below. The maximum current selector 720 enables the user to increase or decrease the maximum amount of stimulation current that can be configured for either mode (i.e., anodic or cathodic) for the selected program (e.g., using the external controller 40). The selected value of 5.0 mA in the illustrated example indicates that the patient cannot increase the total stimulation current above 5.0 mA for either mode for stimulation program 1. The minimum current selector 722 functions in a similar manner to the maximum current selector 720 and enables the user to increase or decrease the minimum amount of stimulation current that can be configured for either mode for the selected program.

The third window 754 includes a stimulation mode selector 724, a current step size selector 726, a current amplitude and electrode allocation selector 728, a therapeutic benefit ranking selector 738, a side effect ranking selector 740, and a notes selector 742. The stimulation mode selector 724 enables the user to select whether anodic or cathodic stimulation is being configured for the selected program. Note that the anodic and cathodic stimulation modes are of opposite polarity (i.e., the current that is issued on the lead-based electrodes is of opposite polarities). In the illustrated example, the stimulation mode selector 724 is selected for configuring cathodic stimulation and thus the IPG 10's case receives 100% of the anodic current and the cathodic current is allocated amongst the lead-based electrodes as specified by the user and as described below. In one embodiment, the stimulation mode selector 724 is configured to default to the anodic stimulation mode when the IPG 10 comprises a rechargeable battery (as a rechargeable battery may be better suited to handle the typically higher energy usage of anodic stimulation) and to default to the cathodic stimulation mode when the IPG 10 comprises a primary cell battery (as a primary cell battery may be less suited to handle the typically higher energy usage of anodic stimulation). The current step size selector 726 enables the user to select the granularity with which the total stimulation current amplitude can be adjusted using the current amplitude and electrode allocation selector 728.

The current amplitude and electrode allocation selector 728 includes a stimulation current amplitude selector 730, an along-lead steering adjuster 736, segmented electrode rotational steering adjusters 732, and segmented electrode focus adjusters 734. The stimulation current amplitude selector 730 enables the user to increase or decrease the total amount of stimulation current that is provided during each pulse. The specified total stimulation current amplitude is adjusted by the number of milliamps specified in the step size selector 726 with each button press. The along-lead steering adjuster 736 enables the user to allocate the total specified stimulation current among the different axial locations along the lead. In one embodiment, the user may select an electrode on the lead representation 710 and the selected electrode will initially be allocated 100% of the stimulation current of the selected mode's polarity. From that original selection, the user can then use the up and down arrows of the along-lead steering adjuster 736 to move a portion of the stimulation current to a different axial location along the lead in the direction of the selected arrow. For example, with electrode E1 selected, a first press of the along-lead steering adjuster 736's up arrow may cause 10% of the stimulation current to be moved from electrode E1 and split equally amongst electrodes E2-E4, which are situated at the axial location along the lead that is directly above electrode E1. Thus, 90% of the cathodic current would be allocated to electrode E1 and 3.33% of the cathodic current would be allocated to each of the electrodes E2 through E4. An additional press of the along-lead steering adjuster 736's up arrow may cause an additional 10% of the stimulation current to be moved from electrode E1 and split equally amongst electrodes E2-E4 such that 80% of the cathodic current would be allocated to electrode E1 and 6.66% of the cathodic current would be allocated to each of electrodes E2 through E4.

The segmented electrode rotational steering adjusters 732 similarly enable the user to steer current between segmented electrodes at the same axial location along the lead in the direction specified by the rotational steering adjusters 732. The segmented electrode focus adjusters 734 enable the user to radially spread or shrink the focus of the stimulation field for a selected set of segmented electrodes. The therapeutic benefit ranking selector 738 enables the user to provide a therapeutic ranking (e.g., on a standard scale of 0-4) for a selected one or more of multiple listed symptoms. The side effect ranking selector 740 enables the user to provide a side effect ranking (e.g., on a standard scale of 0-4) for a selected one or more of multiple listed side effects. The note selector 742 provides a pop-up that enables the user to type a note. The rankings that are entered via the therapeutic benefit and side effect ranking selectors 738 and 740 as well as the notes entered via the note selector 742 are stored in conjunction with the stimulation parameters that were selected at the time the ranking or note was entered. Thus, the rankings and notes can provide an indication of the effectiveness of different stimulation parameters. In one embodiment the rankings and notes can be visualized via another portion of the CP GUI 94'.

The bottom portion of Figure 7 shows an example of the waveforms that are formed at selected electrode E1 for example configurations of the cathodic and anodic stimulation modes for a stimulation program 760. While only the waveform for electrode E1 is shown, it will be understood that the waveform for the IPG 10's case has the same shape and the opposite polarity for both the cathodic and anodic stimulation modes. It will also be understood that although a single selected electrode E1 is shown in the examples, the stimulation current may be allocated amongst one or more of the other lead-based electrodes as described above. In a preferred embodiment, the allocation of current amongst the lead-based electrodes and the case for a particular stimulation program must be the same for both the cathodic and anodic stimulation modes (i.e., the cathodic stimulation mode and the anodic stimulation mode use the same set of electrodes). The example waveform for the anodic stimulation mode 762 comprises a periodic application of a square wave pulse at a frequency f_{A}. The square pulses (each of which may be referred to as an anodic pulse) have an amplitude A_{A} and a pulse width PW_{A}. The example waveform for the cathodic stimulation mode 764 similarly comprises a periodic application of a square wave pulse at a frequency f_{C} that is equal to the frequency f_{A}. In one embodiment, the frequency for a particular stimulation program must be the same for both the cathodic and anodic stimulation modes, but this is not strictly required. The square pulses in the waveform for the cathodic stimulation mode 764 (each of which may be referred to as a cathodic pulse) have an amplitude A_{C} that is smaller than the amplitude A_{A} and a pulse width PW_{C} that is larger than the pulse width PW_{A}. Cathodic stimulation typically requires lower stimulation amplitude than anodic stimulation due to the different biological mechanisms involved in these different modes of stimulation.

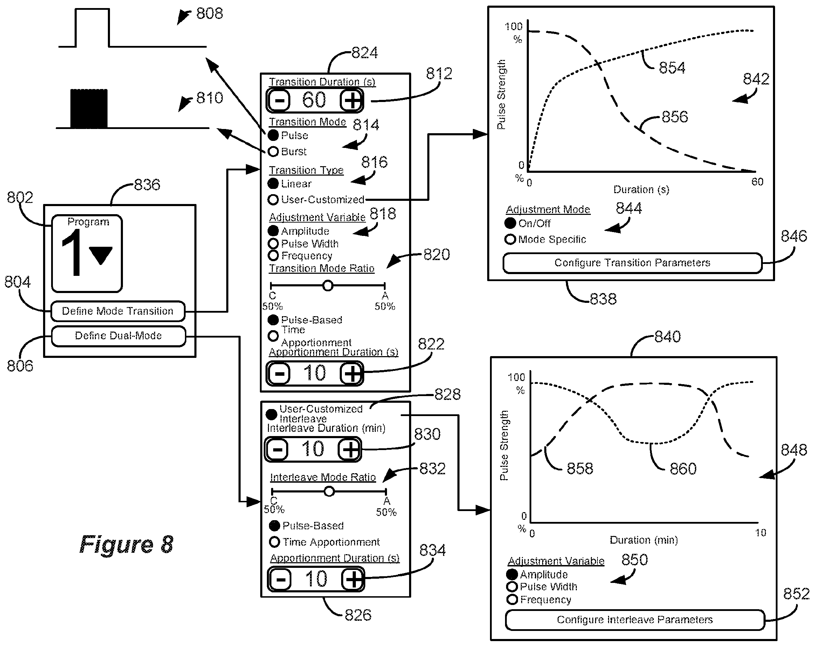

Because the programming interface 700 enables the configuration of both cathodic and anodic stimulation modes each of which may have different beneficial effects, the inventors further recognize that it is beneficial to enable the configuration of the interaction between these two modes when both modes are configured. Figure 8 shows an example of a bimodal definition interface 836 that enables the user to configure the interaction between the cathodic and anodic stimulation modes for each stimulation program in which both modes are configured. The bimodal definition interface 836 includes a program selector 802, a mode transition definition selector 804, and a continuous dual-mode definition selector 806. The program selector 802 functions in the same manner as the program selector 702 in that it enables the user to select the stimulation program for which bimodal stimulation is being defined. In one embodiment, only the stimulation programs for which both cathodic and anodic stimulation modes have been configured are available for selection via the program selector 802.

The mode transition definition selector 804 is a button that enables the user to define the manner in which a transition between the anodic and cathodic stimulation modes (i.e., a first stimulation mode and a second stimulation mode) will take place when such a transition is initiated. Transitions may be initiated manually (e.g., by a user via the external controller 40) or automatically (e.g., based on a closed loop feedback control system or as part of another program that specifies transitions between programs). Although described in terms of a transition between anodic and cathodic stimulation modes, the transition definition selector may also find applicability in transitions of other types such as transitions between two programs of the same polarity or for any number or configuration of programs where the transition manner and timing may be the same, or vary, on a transition by transition basis. Selection of the mode transition definition selector 804 causes a transition definition interface 824 to be displayed. In the illustrated embodiment, the transition definition interface 824 includes a transition duration selector 812, a transition mode selector 814, a transition type selector 816, an adjustment variable selector 818, and a transition mode ratio selector 820. The transition duration selector 812 enables the user to specify the time period over which a requested transition from the configured anodic stimulation mode to the configured cathodic stimulation mode (or vice versa) will occur. In the illustrated embodiment, the transition duration is defined in seconds and a value of 60 seconds is shown. In one embodiment, the transition duration may be configurable between one second and 300 seconds although longer and shorter durations may also be appropriate.

The transition mode selector 814 enables the user to select either pulse mode or burst mode to be implemented during the transition between modes. In pulse mode, the regular pulses (with the amplitude or pulse width modified according to the transition parameters as described below) are utilized during the transition duration as shown in waveform 808. In burst mode, the regular pulses (with the amplitude or pulse width modified according to the transition parameters as described below) are modified such that each pulse is replaced with a high frequency burst of pulses as shown in waveform 810. Although not specifically illustrated, the amplitude of the pulses in a burst may differ from the amplitude of the pulse that the burst replaces such that the amount of charge delivered during the burst is equal to the amount of charge that would have been delivered during the replaced pulse.

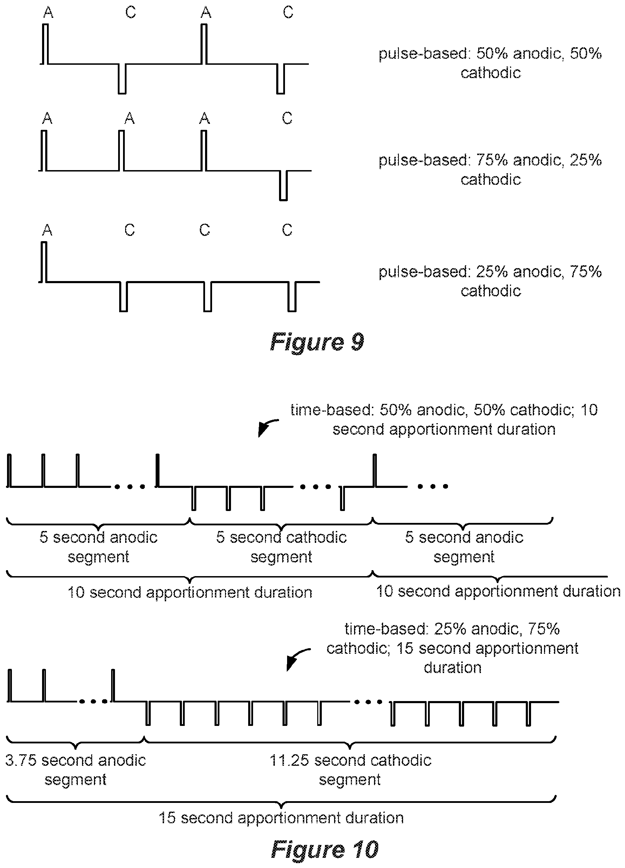

The transition type selector 816 enables the user to select either a linear transition or a user-customized transition. Although not shown in the illustrated example, the transition type selector 816 might also allow the definition of a set of transitions where the clinician may specify that the transitions will be selected from that set in a pseudo random manner. The adjustment variable selector 818 enables the user to select stimulation amplitude, pulse width, or frequency as the variable to be adjusted during the transition period. During the transition period (i.e., the time period following initiation of a stimulation mode change and of the duration specified via the transition duration selector 812), stimulation alternates between the anodic and cathodic pulses, and the apportionment of anodic and cathodic pulses is defined via the transition mode ratio selector 820. The transition mode ratio selector 820 enables the user to specify the time apportionment of each mode during the transition period. For example, when the transition mode ratio is set to 50% anodic and 50% cathodic, half of the pulses during the transition period are anodic pulses and half of the pulses are cathodic pulses. Similarly, when the transition mode ratio is set to 75% anodic and 25% cathodic, 75% of the pulses during the transition period are anodic pulses and 25% the pulses are cathodic pulses. The user can customize the apportionment of stimulation modes as either pulse-based or time-based using the selectors illustrated in the transition mode ratio selector 820. The differences between these apportionment types are illustrated in Figures 9 and 10.

Figure 9 illustrates three examples of pulse-based mode apportionment. In pulse-based mode apportionment, apportionment occurs on a small time scale such that the selected apportionment is enforced even over a small number of pulses. The top portion of Figure 9 shows an example of a pulse-based apportionment for a selected ratio of 50% anodic mode and 50% cathodic mode. As shown, anodic and cathodic pulses are interleaved with a single anodic pulse followed by a single cathodic pulse. The middle portion of Figure 9 shows an example of a pulse-based apportionment for a selected ratio of 75% anodic mode and 25% cathodic mode. As shown, anodic and cathodic pulses are interleaved with three anodic pulses followed by a single cathodic pulse. The bottom portion of Figure 9 shows an example of a pulse-based apportionment for a selected ratio of 25% anodic mode and 75% cathodic mode. As shown, anodic and cathodic pulses are interleaved with a single anodic pulse followed by three cathodic pulses. When pulse-based apportionment is selected, the transition mode ratio selector 820 may only enable the selection of ratios that can be enforced over a relatively small number of pulses (e.g., eight pulses).

Figure 10 illustrates two examples of time-based apportionment, which occurs over a longer time interval than pulse-based apportionment. In time-based mode apportionment, the user specifies an apportionment duration (e.g., using the apportionment duration selector 822), and apportionment periods of the specified duration are divided into segments in accordance with the relative apportionment selected via the transition mode ratio selector 820. Pulses of a particular mode are applied during the corresponding segment of the apportionment duration, and apportionment durations are repeated in succession. For example, the top portion of Figure 10 shows an example of a time-based apportionment for a selected ratio of 50% anodic mode and 50% cathodic mode with an apportionment duration of ten seconds. As shown, anodic pulses are applied during a five second anodic segment of the apportionment duration and cathodic pulses are applied during a five second cathodic segment of the apportionment duration. This is repeated in a subsequent ten second apportionment duration. The bottom portion of Figure 10 shows an example of a time-based apportionment for a selected ratio of 25% anodic mode and 75% cathodic mode with an apportionment duration of 15 seconds. As shown, anodic pulses are applied during a 3.75 second anodic segment of the apportionment duration and cathodic pulses are applied during a 11.25 second cathodic segment of the apportionment duration. This is repeated in a subsequent 15 second apportionment duration (not shown). When time-based apportionment is selected, it is preferred that the selected apportionment duration is much shorter than the specified transition duration such that pulses of each type are applied during numerous apportionment periods during the transition period. Note that the transition mode ratio is only effective during a transition period and does not otherwise impact stimulation.

Returning to Figure 8, when the linear transition type is selected via the transition type selector 816, the amount of charge per pulse for the currently-active stimulation mode is decreased from that specified in the programming interface 700 to zero and the amount of charge per pulse for the currently-inactive stimulation mode is increased from zero to that specified in the programming interface 700 during the transition period. The charge per pulse is increased or decreased by adjusting the adjustment variable (i.e., amplitude, pulse width, or frequency) that is specified via the adjustment variable selector 818. Pulses of the currently-active stimulation mode type and the currently-inactive stimulation mode type are alternated according to the transition mode ratio settings during the transition period, and at the end of the transition period all pulses are of the transitioned-to mode.

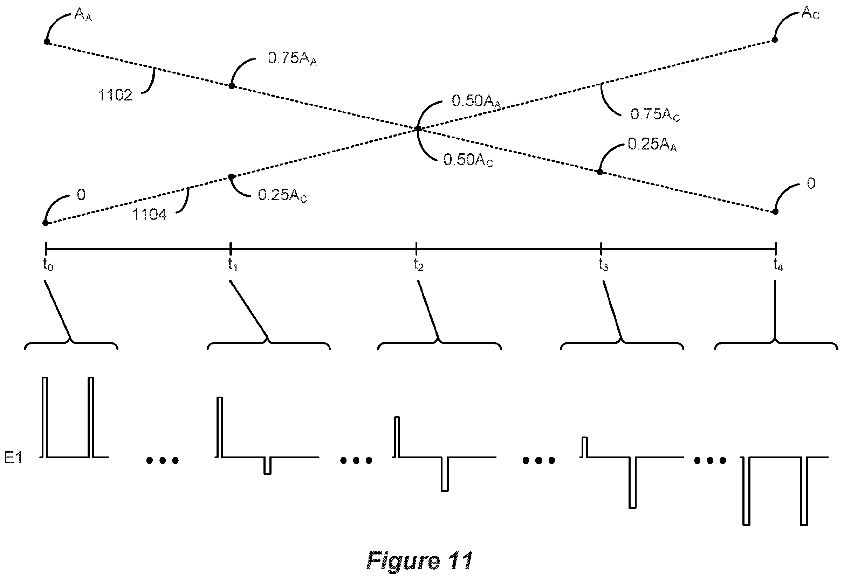

Figure 11 illustrates an example of a waveform that is generated at electrode E1 for a linear transition from the anodic stimulation mode 762 to the cathodic stimulation mode. In the example shown, the selected adjustment variable is stimulation amplitude, the transition mode ratio is set to 50% anodic and 50% cathodic, and the mode ratio type is set to pulse-based. Given that the adjustment variable is amplitude, anodic pulses decrease linearly in amplitude from A_{A} to zero during the transition period and cathodic pulses increase linearly in amplitude from zero to A_{C} during the transition period. The decrease in anodic stimulation amplitude is illustrated by the line 1102 and the increase in cathodic stimulation amplitude is illustrated by the line 1104. At time t₀, stimulation is being delivered in the anodic mode of operation and only anodic pulses are issued at the configured settings (A_{A} and PW_{A}). A transition from anodic to cathodic mode is initiated at t₀, and thus the amplitudes of anodic stimulation pulses begin to decrease and the amplitudes of cathodic stimulation pulses begin to increase. At time t₁, anodic and cathodic pulses are interleaved according to the transition mode ratio settings and anodic pulses are issued at 75% of the configured amplitude and the configured pulse width (0.75A_{A} and PW_{A}) and cathodic pulses are issued at 25% of the configured amplitude and the configured pulse width (0.25A_{C} and PW_{C}). At time t₂, anodic and cathodic pulses are interleaved according to the transition mode ratio settings and anodic pulses are issued at 50% of the configured amplitude and the configured pulse width (0.50A_{A} and PW_{A}) and cathodic pulses are issued at 50% of the configured amplitude and the configured pulse width (0.50A_{C} and PW_{C}). At time t₃, anodic and cathodic pulses are interleaved according to the transition mode ratio settings and anodic pulses are issued at 25% of the configured amplitude and the configured pulse width (0.25A_{A} and PW_{A}) and cathodic pulses are issued at 75% of the configured amplitude and the configured pulse width (0.75A_{C} and PW_{C}). At time t₄, the transition to the cathodic stimulation mode is complete and only cathodic pulses are issued at the configured settings (A_{C} and PW_{C}).

Throughout the transition period, the pulses, whether anodic or cathodic, are applied at the frequency that is defined via the programming interface 700, which is the same for both stimulation modes (i.e., f_{A} = f_{C}). Because the adjustment variable is amplitude, the pulse width for each pulse is as specified via the programming interface 700 (i.e., PW_{A} for the anodic pulses and PW_{C} for the cathodic pulses). As will be understood, if pulse width is alternatively selected as the adjustment variable, the amplitude for each pulse during the transition period would be as specified via the programming interface 700 (i.e., A_{A} for the anodic pulses and A_{C} for the cathodic pulses) and the pulse width would decrease from PW_{A} to zero for the anodic pulses and increase from zero to PW_{C} for the cathodic pulses over the transition period.

Returning to Figure 8, if the user selects the user-customized transition type via the transition type selector 816 a user-customized transition interface 838 is displayed. The user customized transition interface 838 includes a transition plot 842, an adjustment mode selector 844, and a configure transition parameters selector 846. The configure transition parameters selector 846 enables the user to define a set of pulse strength (i.e., the amount of charge per pulse as a proportion of the configured amount of charge per pulse or, more specifically, the proportion of the configured value of the adjustment variable) values each corresponding to a different time during the transition duration for different mode transitions. Because the points define a transition between the two modes, the pulse strength values at the transition duration endpoints (e.g., zero seconds and 60 seconds in the example shown) are fixed with one endpoint fixed at 100% of the configured pulse strength and the other endpoint fixed at 0% of the configured pulse strength. One set of pulse strength values specifies a proportion as increasing from zero to unity and another set of values specifies the proportion as decreasing from unity to zero In one embodiment, the user may select the pulse strength values at multiple times between the transition duration endpoints on a plot such as the transition plot 842. In another embodiment, the user may enter pulse strength value/time value pairs for time values between the transition duration endpoints via an entry field. The user may further select a fitting technique such as a linear fitting technique or a curve fitting technique that represents the configured points as a function of time. Having specified a number of points and a fitting technique, the configured transitions are plotted in the transition plot 842 as pulse strength (in terms of percent of configured pulse strength) as a function of time over the transition period (i.e., the duration selected via the transition duration selector 812). In the illustrated embodiment, two example user-configured transitions are illustrated: an on to off transition that is illustrated by the on/off curve 856 and an off to on transition that is illustrated by the off/on curve 854. As can be seen, the on/off curve 856 decreases the pulse strength of the currently-active stimulation mode gradually over a first portion of the transition period, then quickly during a middle portion of the transition period, and then again gradually to zero over an end portion of the transition period. The off/on curve 854 increases the pulse strength of the currently-inactive stimulation mode quickly over a beginning portion of the transition period then less quickly over the remaining portion of the transition period.

The adjustment mode selector 844 enables the user to specify either an on/off mode or a mode-specific mode. In the on/off mode, which is selected in the illustrated example, the user can define an on to off transition and an off to on transition such as those illustrated in the transition plot 842 in the illustrated example. When a transition between modes is initiated in the on/off mode, the currently-active stimulation mode transitions according to the configured on to off transition and the currently-inactive stimulation mode transitions according to the configured off to on transition regardless of which stimulation mode is active and which is inactive. In the mode-specific mode, the user can define an on to off transition and an off to on transition for each stimulation mode. When a transition between modes is initiated in the mode-specific mode, the currently-active stimulation mode transitions according to its specific on to off transition and the currently-inactive stimulation mode transitions according to its specific off to on transition. The mode-specific mode thus enables the user to configure different settings for a cathodic to anodic transition and an anodic to cathodic transition.

In another embodiment, the user may be able to specify a function over the duration of the transition period similar to the curve 854 or 856 where the function specifies a probability of which transition mode will be used at each particular point in time during the transition. In such an embodiment, a pseudo random generator may be utilized to determine, based on the probability defined by the curve, the particular mode that will be utilized at any particular point in time. In a similar embodiment, the user may be able to specify a function over the duration of the interleave period where the function specifies a probability of which transition curve (i.e., 854 or 856) will be active at a given time. In such an embodiment, a pseudo random generator may be utilized to determine, based on the probability defined by the probability curve, the transition curve that will be utilized, and stimulation will be based on the mode corresponding to the selected transition curve as adjusted by the selected transition curve. In yet another embodiment, a transition may be defined by a single transition curve and one of the stimulation modes may be based on the values in the curve and the other of the stimulation modes may be based on the opposite of the value in the curve (i.e., curve specifies a proportion of 0.4, which applies to a first stimulation mode and an opposite proportion, 0.6, applies to the second stimulation mode). Regardless of the manner in which a transition is defined by the user, stimulation parameters that specify the parameters of the stimulation are communicated to the IPG 10.

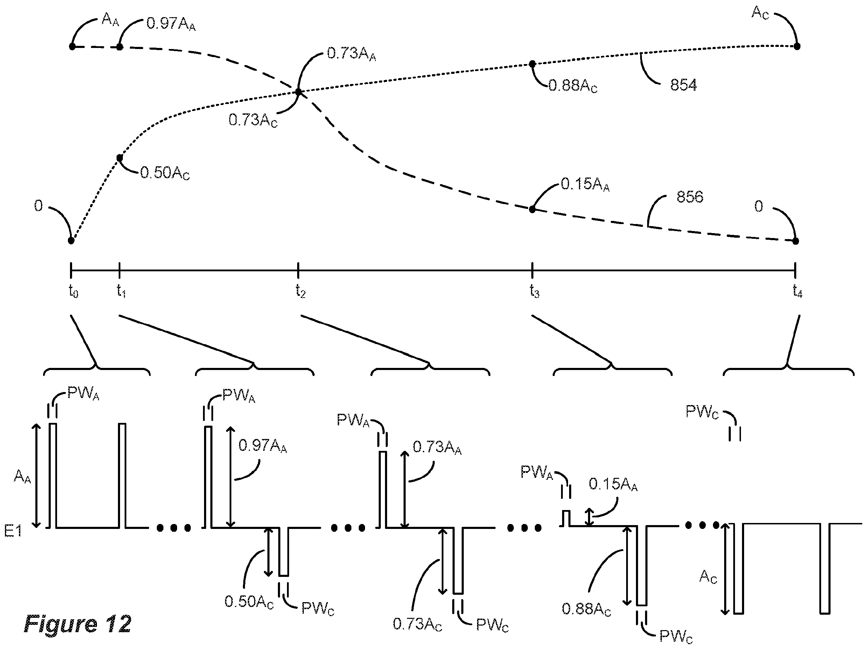

Figure 12 illustrates an example of a waveform that is generated at electrode E1 for a user-configured transition from the anodic stimulation mode 762 to the cathodic stimulation mode 764 using the example transition parameters that are shown in the user-customized transition interface 838 in Figure 8. In the example shown, the selected adjustment variable is stimulation amplitude, the transition mode ratio is set to 50% anodic and 50% cathodic, and the transition mode ratio type is pulse-based. Given that the adjustment variable is amplitude, anodic pulses decrease in amplitude from A_{A} to zero according to the on/off curve 856 during the transition period and cathodic pulses increase in amplitude from zero to A_{C} according to the off/on curve 854 during the transition period. As shown in the example waveform, anodic and cathodic pulses are interleaved during the transition period according to the transition mode ratio settings. The anodic and cathodic pulses are applied at the frequency that is defined via the programming interface 700, which is the same for both stimulation modes (i.e., f_{A} = f_{C}).

At time t₀, the anodic stimulation mode 762 is selected and anodic pulses are being applied according to the parameters specified via the programming interface 700 for the anodic stimulation mode (i.e., applied at a frequency f_{A} with a pulse width PW_{A} and an amplitude A_{A}). At time t₀, a transition from the anodic stimulation mode 762 to the cathodic stimulation mode 764 is initiated. At time t₁, the transition from anodic stimulation to cathodic stimulation is ongoing and the on/off transition curve 856 indicates that anodic pulses shall be at 97% of the per-pulse charge that is specified via the programming interface 700 and the off/on transition curve 854 indicates that cathodic pulses shall be at 50% of the per-pulse charge that is specified via the programming interface 700. Because the adjustment variable is amplitude, this indicates that anodic pulses are issued at 97% of the configured amplitude and at the configured pulse width (i.e., 0.97A_{A} and PW_{A}) and cathodic pulses are issued at 50% of the configured amplitude and at the configured pulse width (i.e., 0.50A_{C} and PW_{C}). As shown in the example waveform, anodic and cathodic pulses having the specified parameters are interleaved at time t₁ according to the transition mode ratio settings. At time t₂, the on/off transition curve 856 indicates that anodic pulses shall be at 73% of the configured per-pulse charge and the off/on transition curve 854 indicates that cathodic pulses shall be at 73% of the per-pulse charge, so both anodic pulses and cathodic pulses are issued at 73% of the configured amplitudes and at the configured pulse widths (i.e., 0.73A_{A} and PW_{A} for anodic pulses and 0.73A_{C} and PW_{C} for cathodic pulses). As shown in the example waveform, anodic and cathodic pulses having the specified parameters are interleaved at time t₂ according to the transition mode ratio settings. Note that although both anodic and cathodic pulses are issued at 73% of their configured amplitudes at time t₂, the anodic pulses have a higher amplitude due to their higher configured amplitude value. Similarly, although both anodic and cathodic pulses are issued at their configured pulse widths, the cathodic pulses have a longer pulse width due to their longer configured pulse width value. At time t₃, the on/off transition curve 856 indicates that anodic pulses shall be at 15% of the configured per-pulse charge and the off/on transition curve 854 indicates that cathodic pulses shall be at 88% of the configured per-pulse charge, so anodic pulses are issued at 15% of the configured amplitude and at the configured pulse width (i.e., 0.15A_{A} and PW_{A}) and cathodic pulses are issued at 88% of the configured amplitude and at the configured pulse width (i.e., 0.88A_{C} and PW_{C}). As shown in the example waveform, anodic and cathodic pulses having the specified parameters are interleaved at time t₃ according to the transition mode ratio settings. At time t₄, the transition is complete and cathodic pulses are being applied according to the parameters specified via the programming interface 700 for the cathodic stimulation mode 764 (i.e., applied at a frequency f_{C} with a pulse width PW_{C} and an amplitude A_{C}) with no anodic pulses being applied.

As will be understood, if pulse width is alternatively selected as the adjustment variable, the amplitude for each pulse during the transition period would be as specified via the programming interface 700 (i.e., A_{A} for the anodic pulses and A_{C} for the cathodic pulses) and the pulse width would decrease from PW_{A} to zero according to the on/off curve 856 for the anodic pulses and increase from zero to PW_{C} according to the off/on curve 854 for the cathodic pulses over the transition period. As can be understood from the examples, the mode transition definition interface 824 gives the user precise control over transitions between configured stimulation modes.

Returning to Figure 8, having described the configurability of mode transitions via the mode transition definition selector 804, the configurability of continuous dual-mode operation via the continuous dual-mode definition selector 806 of the bimodal definition interface 836 is now described. The continuous dual-mode definition selector 806 is a button that enables the user to define continuous dual-mode stimulation that includes both the anodic and cathodic stimulation modes. Dual-mode operation differs from the above-described transitions in that dual-mode operation comprises the continuous use of both the anodic and cathodic stimulation modes rather than a transition between the stimulation modes. Selection of the continuous dual-mode definition selector 806 causes a dual-mode definition interface 826 to be displayed. In the illustrated embodiment, the dual-mode definition interface 826 includes a user-customized interleave selector 828, an interleave duration selector 830, an interleave mode ratio selector 832, and an apportionment duration selector 834. The interleave duration selector 830 enables the user to specify the time period over which a user-customized interleaving of the anodic and cathodic stimulation modes is repeated. In the illustrated embodiment, the interleave duration is defined in minutes and a value of 10 minutes is shown. In one embodiment, the interleave duration may be configurable between one minute and 180 minutes although longer and shorter durations may also be appropriate.

When the user selects the user-customized interleave selector 828, a user-customized interleave interface 840 is displayed. The user-customized interleave interface 840 includes an interleave plot 848, an adjustment variable selector 850, and a configure interleave parameters selector 852. The user-customized interleave interface 840 functions in a similar manner to the user-customized transition interface 838 in that it enables the user to define pulse strength at multiple times during the interleave duration. The interleave interface 840 differs from the transition interface 838 in that the interleave duration is repeated whereas the transition duration occurs only once upon the initiation of a mode transition. The interleave interface 840 further differs from the transition interface 838 in that the pulse strength is not fixed at 100% and 0% at the boundaries of the interleave duration as is the case at the boundaries of the transition duration. Thus, the user can define the pulse strength for both the anodic stimulation mode and the cathodic stimulation mode at any point in time during the interleave duration. The specified pulse strength values for the anodic and cathodic stimulation modes are sets of inputs that specify adjustments to a parameter of the anodic and cathodic stimulation modes over the interleave duration. The user may select the pulse strength values for each of the cathodic and anodic stimulation modes at time points on a plot such as the interleave plot 848 or enter pulse strength value/ time value pairs via an entry field. The user may further select a fitting technique such as a linear fitting technique or a curve fitting technique that represents the configured points as a function of time. Having specified a number of points and a fitting technique, the configured mode strength functions are plotted in the interleave plot 848 as pulse strength (in terms of percent of configured pulse strength) as a function of time over the interleave duration (i.e., the duration selected via the interleave duration selector 830). In the illustrated embodiment, the configured mode strength functions are illustrated as an anodic mode strength curve 860 and a cathodic mode strength curve 858. As can be seen, the cathodic mode strength curve 858 increases from an initial value of approximately 50% at the beginning of the interleave duration to a maximum of 100% at approximately the middle of the interleave duration and then decreases back to a value of approximately 50% at the end of the interleave duration. The anodic mode strength curve 860 behaves in an essentially opposite manner as it decreases from an initial value of 100% at the beginning of the interleave duration to a value of approximately 58% at approximately the middle of the interleave duration and then increases back to a value of approximately 100% at the end of the interleave duration. Although the illustrated anodic mode strength curve 860 and the cathodic mode strength curve 858 behave in similar but opposite manners, this is not a requirement and each curve can be configured in any desired manner within the programming limits of the IPG 10. Moreover, although the illustrated mode strength curves are of substantially the same value at the beginning and end of the interleave duration, this is also not required although a curve with different beginning and ending values would result in a sudden change in the stimulation parameters for the particular mode defined by the curve each time the interleave duration repeated. Still further, although the illustrated mode strength curves do not exceed 100% during the interleave duration, there is no prohibition on such a configuration as long as the defined adjustment would not result in stimulation that exceeds a defined limit (e.g., the defined maximum stimulation current limit).

The adjustment variable selector 850 functions in the same manner as the adjustment variable selector 818 in that it enables the user to specify whether stimulation amplitude or pulse width is adjusted to adjust the pulse strength in accordance with the configured mode strength functions. The interleave mode ratio selector 832 functions in the same manner as the transition mode ratio selector 820 in that it allows the user to specify the time apportionment of the cathodic and anodic stimulation modes and to select pulse-based or time-based allocation of the different stimulation modes. However, as noted above, the parameters that are specified via the dual-mode definition interface 826 are continuous parameters, and therefore the mode ratio specified via the interleave mode ratio selector 820 specifies a continuous interleaving of the stimulation modes according to the selected settings whereas the transition mode ratio selector 820 simply defines the interleaving of the stimulation modes during a transition period. The interleave mode ratio selector 820 can be used alone to select the apportionment of the cathodic and anodic stimulation modes to be applied as configured via the programming interface 700 (i.e., without modification via user-customized interleaving).

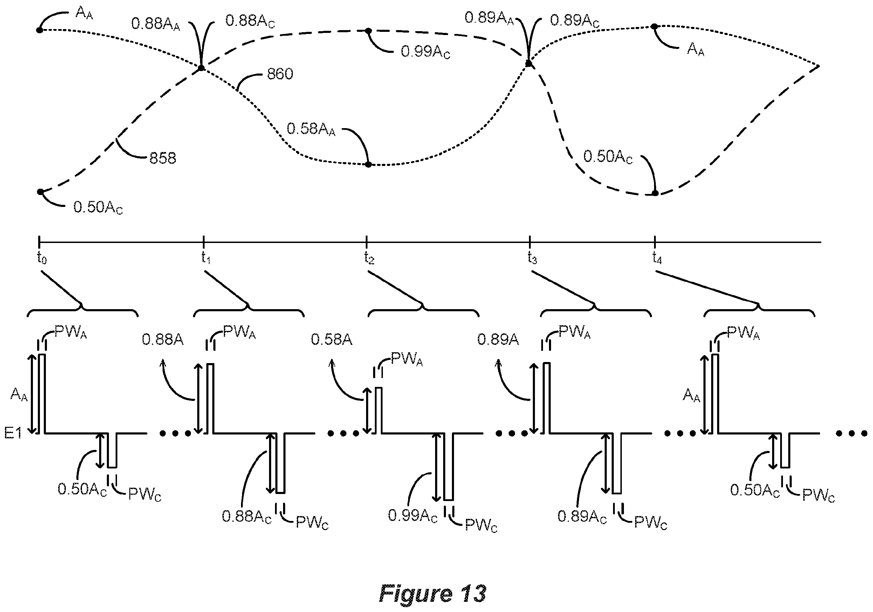

Figure 13 illustrates an example of a waveform that is generated at electrode E1 for continuous user-customized interleaving of the anodic stimulation mode 762 and the cathodic stimulation mode 764 using the example user-customized interleaving parameters that are shown in the user-customized interleave interface 840 in Figure 8. In the example shown, the selected adjustment variable is stimulation amplitude, the mode ratio is set to 50% anodic and 50% cathodic, and the mode ratio type is pulse-based. Given that the adjustment variable is amplitude, the amplitudes of the anodic and cathodic pulses are determined based on the values of the anodic mode strength function 860 and the cathodic mode strength function 858, respectively, at a given time during the repeated interleave duration. As shown in the example waveform, anodic and cathodic pulses are interleaved with a single anodic pulse followed by a single cathodic pulse in accordance with the mode ratio settings. The anodic and cathodic pulses are applied at the frequency that is defined via the programming interface 700, which is the same for both stimulation modes (i.e., f_{A} = f_{C}).

Time t₀ corresponds to the beginning of the interleave period, at which point the anodic mode strength curve 860 indicates that anodic pulses shall be at 100% of the per-pulse charge that is specified via the programming interface 700 and the cathodic mode strength curve 858 indicates that cathodic pulses shall be at 50% of the per-pulse charge that is specified via the programming interface 700. Thus, at time t₁, anodic pulses are issued at the configured amplitude and pulse width (i.e., A_{A} and PW_{A}) and cathodic pulses are issued at 50% of the configured amplitude and at the configured pulse width (i.e., 0.50A_{C} and PW_{C}). As shown in the example waveform, anodic and cathodic pulses having the specified parameters are interleaved at time t₀ according to the specified interleave mode ratio settings. At time t₁, the anodic mode strength curve 860 indicates that anodic pulses shall be at 88% of the configured per-pulse charge and the cathodic mode strength curve 858 indicates that cathodic pulses shall be at 88% of the configured per-pulse charge, so both anodic pulses and cathodic pulses are issued at 88% of the configured amplitudes and at the configured pulse widths (i.e., 0.88A_{A} and PW_{A} for anodic pulses and 0.88A_{C} and PW_{C} for cathodic pulses). At time t₂, the anodic mode strength curve 860 indicates that anodic pulses shall be at 58% of the configured per-pulse charge and the cathodic mode strength curve 858 indicates that cathodic pulses shall be at 99% of the per-pulse charge, so anodic pulses are issued at 58% of the configured amplitude and at the configured pulse width (i.e., 0.58A_{A} and PW_{A}) and cathodic pulses are issued at 99% of the configured amplitude and at the configured pulse width (i.e., 0.99A_{C} and PW_{C}). At time t₃, the anodic mode strength curve 860 indicates that anodic pulses shall be at 89% of the configured per-pulse charge and the cathodic mode strength curve 858 indicates that cathodic pulses shall be at 89% of the configured per-pulse charge, so both anodic pulses and cathodic pulses are issued at 89% of the configured amplitudes and at the configured pulse widths (i.e., 0.89A_{A} and PW_{A} for anodic pulses and 0.89A_{C} and PW_{C} for cathodic pulses). Like time t₀, time t₄ corresponds to the beginning of the interleave period, which repeats following the completion of the previous interleave period. Thus, at time t₄, just as at time t₀, anodic pulses are issued at the configured amplitude and at the configured pulse width (i.e., A_{A} and PW_{A}) and cathodic pulses are issued at 50% of the configured amplitude and at the configured pulse width (i.e., 0.50A_{C} and PW_{C}) with anodic and cathodic pulses interleaved according to the interleave mode ratio settings.

As can be seen from the illustrated examples, the bimodal definition interface 836 enables sophisticated user configurability of the execution of configured anodic and cathodic stimulation modes. The bimodal definition interface 836 enables the user to precisely define transitions between stimulation modes, to allocate the amount of time spent in each of the stimulation modes, and to precisely configure cyclical adjustments to the configured stimulation modes.

In another embodiment, the user may be able to specify a cyclical function over the duration of the interleave period where the function specifies a probability of which stimulation mode will be used at each particular point in time. In such an embodiment, a pseudo random generator may be utilized to determine, based on the probability defined by the curve, the particular stimulation mode that will be utilized at any particular point in time. In a similar embodiment, the user may be able to specify a cyclical function over the duration of the interleave period where the function specifies a probability of which mode curve (i.e., 858 or 860) will be active at a given time. In such an embodiment, a pseudo random generator may be utilized to determine, based on the probability defined by the probability curve, the mode curve that will be utilized, and stimulation will be based on the mode corresponding to the selected mode curve as adjusted by the selected mode curve. Regardless of the manner in which continuous interleaving is defined by the user, stimulation parameters that specify the parameters of the stimulation are communicated to the IPG 10.

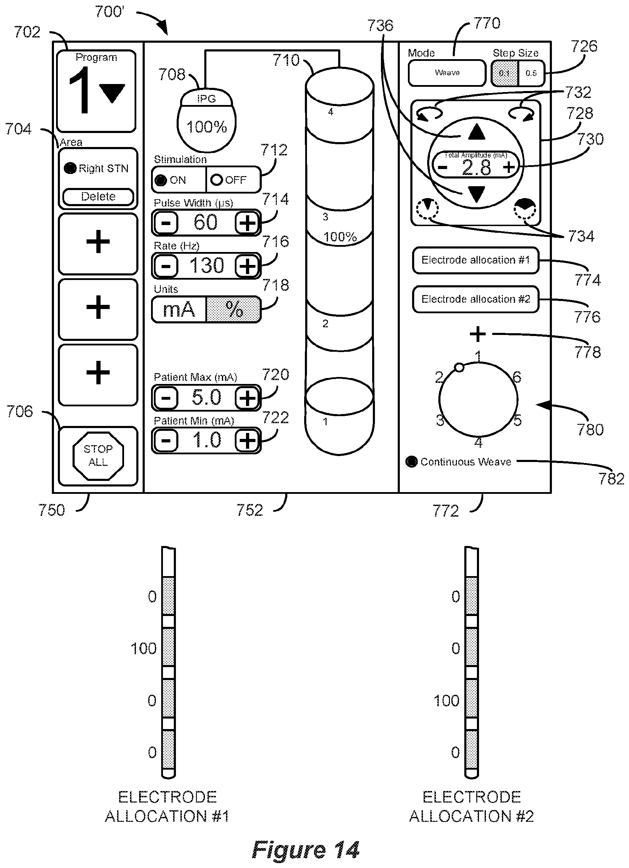

Figure 14 shows an example of a programming interface 700' that enables the user to configure anodic and cathodic stimulation via a different approach. The programming interface 700' includes a first window 750, a second window 752, and a third window 772. The first window 750 and the second window 752 are identical to the corresponding windows of the programming interface 700. The third window 772 differs from the third window 754 of the programming interface 700 in that it enables stimulation to be configured using a weave configuration mode as described below. In the programming interface 700', the mode selector 724 is replaced by a weave mode indicator 770, which indicates that configuration is in the weave configuration mode as opposed to either the anodic or cathodic configuration modes that might be specified by the mode selector 724 as described above. In the weave configuration mode, the user configures the parameters of stimulation such as pulse width, frequency, and stimulation amplitude in the same manner as described above with respect to programming interface 700. However, the user does not directly specify the parameters of separate anodic and cathodic stimulation modes in the manner that is done via the programming interface 700 as described above. Rather, in the weave configuration mode, the user specifies two or more electrode allocations and the CP software 96' defines stimulation regimes based on the specified two or more electrode allocations.

The two or more electrode allocations can be configured by steering the allocation of current amongst the available lead-based electrodes in the same manner as described above with respect to programming interface 700. Specifically, the user can steer the allocation of current amongst the lead-based electrodes using the along-lead steering adjuster 736, segmented electrode rotational steering adjusters 732, and segmented electrode focus adjusters 734 as described above until the desired electrode allocation is indicated on the lead representation 710. Note that for purposes of introducing the weave configuration concept, the lead representation 710 that is depicted in the example programming interface 700' shown in Figure 14 does not include segmented electrodes and instead simply includes four circumferential electrodes at different axial locations along the lead. While this simple example is employed to introduce the weave configuration concept, weave configuration is not limited to circumferential electrodes but can also be employed when a lead having segmented electrodes is used.

When the user has specified a desired first electrode allocation, the first electrode allocation selector 774 is selected to save that electrode allocation as the first electrode allocation. Similarly, when the user has specified the desired second electrode allocation, the second electrode allocation selector 776 is selected to save that electrode allocation as the second electrode allocation. If the user wishes to configure more than two electrode allocations, the add allocation selector 778 can be selected to add an additional electrode allocation selector. The additional electrode allocation can then be saved in the same manner as the first and second electrode allocations by configuring the desired electrode allocation and then selecting the added electrode allocation selector. Further electrode allocations can be added by using the add allocation selector 770 in the same manner. It should be noted that because stimulation is not being defined for a particular stimulation mode such as anodic or cathodic when configuration is performed using the weave configuration mode, the specified electrode allocations are not of a particular polarity but rather simply specify the allocation of the total stimulation current amongst the lead-based electrodes. It should further be noted that the stimulation parameters such as pulse width, frequency, and stimulation current are not associated with the saved electrode allocations. Thus, any changes to these stimulation parameters apply to the selected stimulation program as a whole and not to any particular electrode allocation.

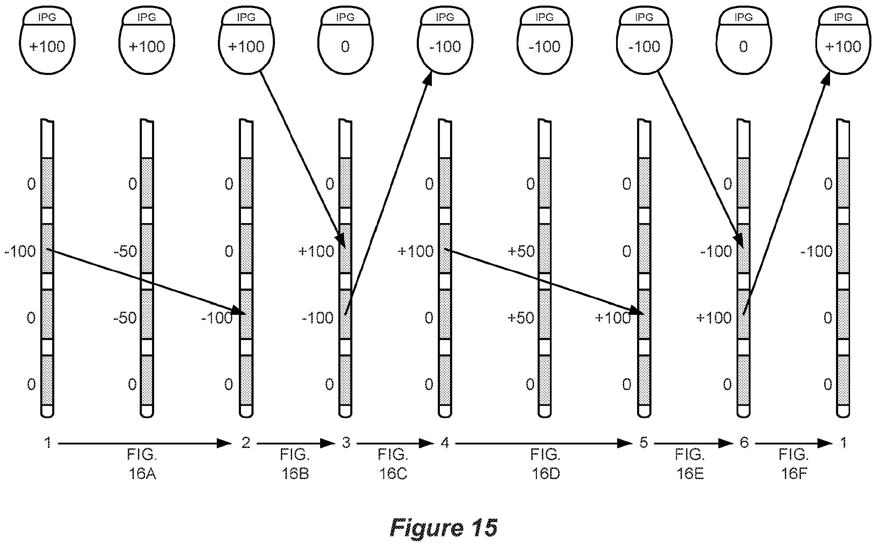

In the illustrated example, the user has defined two electrode allocations, which are shown at the bottom of Figure 14. In the first electrode allocation, 100% of the current is allocated to electrode E3. In the second electrode allocation, 100% of the current is allocated to electrode E2. While these simple examples in which all of the current is allocated to a single lead-based electrode are used to introduce the weave concept, it should be understood that more complex electrode allocations may split the allocation of current amongst multiple lead-based electrodes. Based on the two selected electrode allocations, the CP software 96' defines six different stimulation regimes, which are shown in Figure 15.

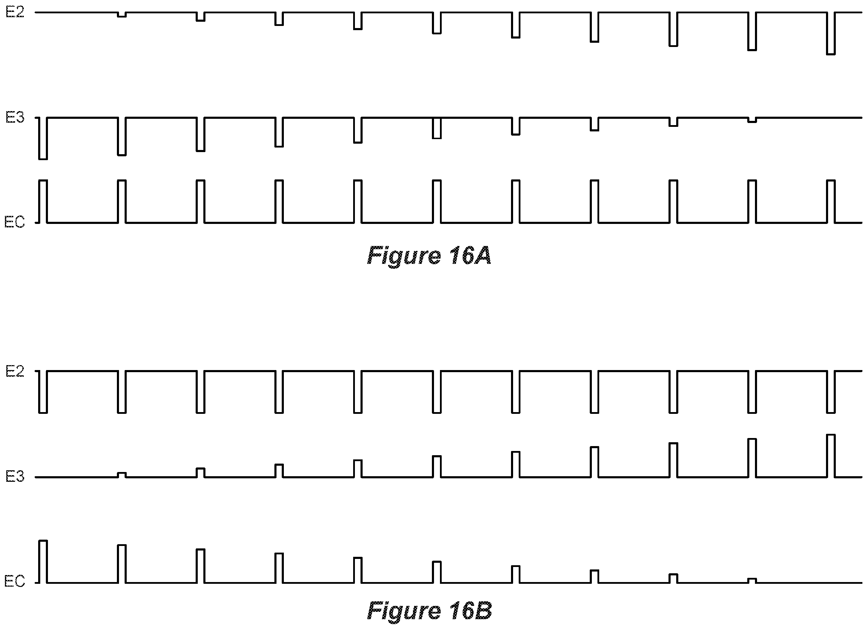

As illustrated in Figure 15, in the first stimulation regime, the first electrode allocation (electrode E3) receives all of the cathodic current and the case receives all of the anodic current. Thus, the first stimulation regime specifies a cathodic stimulation mode. In the second stimulation regime, the second electrode allocation (electrode E2) receives all of the cathodic current and the case (electrode EC) receives all of the anodic current. Thus, like the first stimulation regime, the second stimulation regime specifies a cathodic stimulation mode. Figure 16A shows example waveforms that are formed at electrodes E2, E3, and EC during the transition between the first regime and the second regime. As can be seen, cathodic current is gradually steered from electrode E3 to electrode E2 while the full allocation of anodic current remains constant at the case throughout the transition.

Returning to Figure 15, in the third stimulation regime, the first electrode allocation (electrode E3) receives all of the anodic current and the second electrode allocation (electrode E2) receives all of the cathodic current. This third stimulation regime marks a shift from a cathodic stimulation mode to a bipolar stimulation mode in which all of the stimulation current is directed to lead-based electrodes. Figure 16B shows example waveforms that are formed at electrodes E2, E3, and EC during the transition between the second regime and the third regime. As can be seen, anodic current is gradually steered from the case to electrode E3 while the full allocation of cathodic current remains constant at electrode E2 throughout the transition.

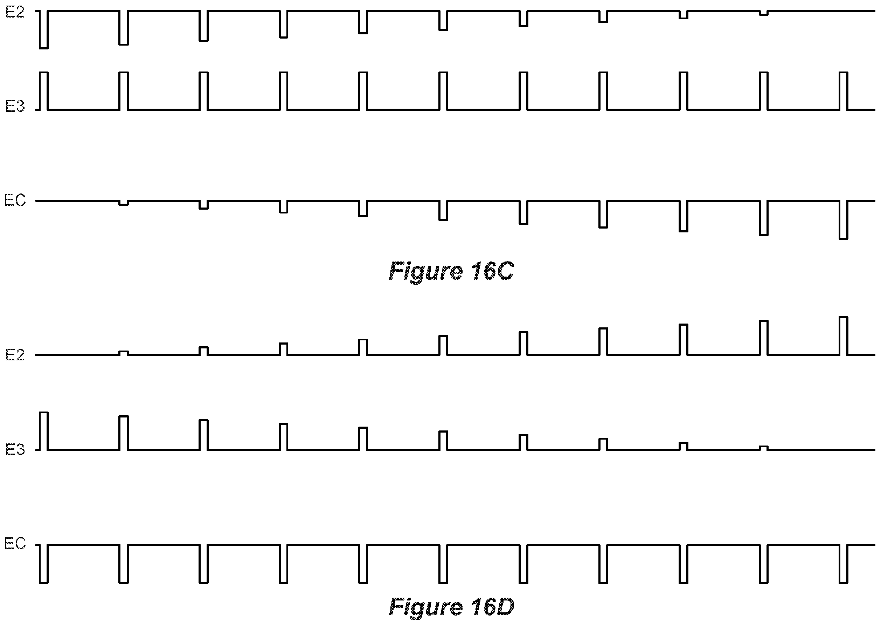

Returning to Figure 15, in the fourth stimulation regime, the first electrode allocation (electrode E3) receives all of the anodic current and the case receives all of the cathodic current. This fourth stimulation regime marks a shift from a bipolar stimulation mode to an anodic stimulation mode. Figure 16C shows example waveforms that are formed at electrodes E2, E3, and EC during the transition between the third regime and the fourth regime. As can be seen, cathodic current is gradually steered from electrode E2 to the case while the full allocation of anodic current remains constant at electrode E3 throughout the transition.

Returning to Figure 15, in the fifth stimulation regime, the second electrode allocation (electrode E2) receives all of the anodic current and the case receives all of the cathodic current. Like the fourth stimulation regime, the fifth stimulation regime specifies an anodic stimulation mode. Figure 16D shows example waveforms that are formed at electrodes E2, E3, and EC during the transition between the fourth regime and the fifth regime. As can be seen, anodic current is gradually steered from electrode E3 to electrode E2 while the full allocation of cathodic current remains constant at the case throughout the transition.

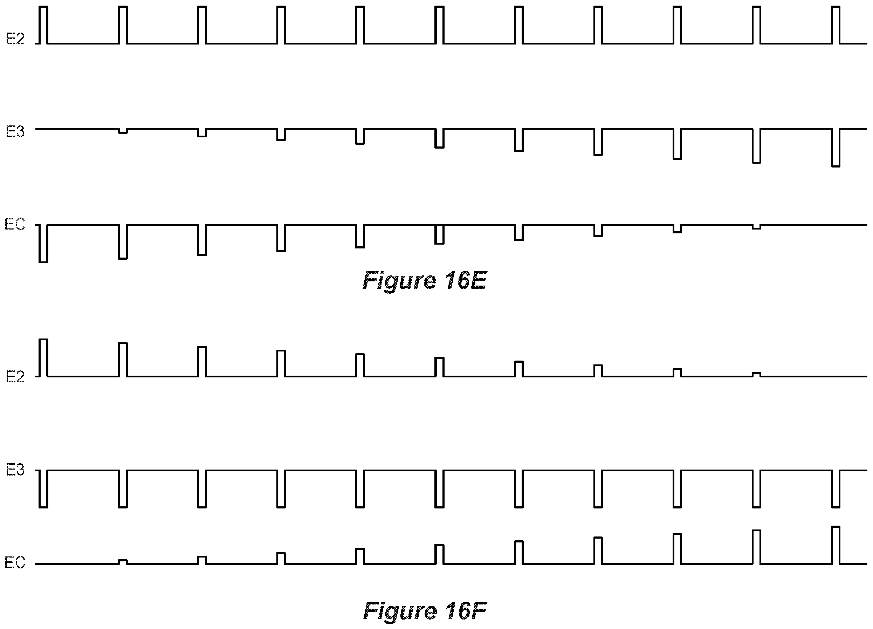

Returning to Figure 15, in the sixth stimulation regime, the first electrode allocation (electrode E3) receives all of the cathodic current and the second electrode allocation (electrode E2) receives all of the anodic current. The sixth stimulation regime marks a shift from an anodic stimulation mode to a bipolar stimulation mode. Figure 16E shows example waveforms that are formed at electrodes E2, E3, and EC during the transition between the fifth regime and the sixth regime. As can be seen, cathodic current is gradually steered from the case to electrode E3 while the full allocation of anodic current remains constant at electrode E2 throughout the transition.

Returning to Figure 15, from the sixth stimulation regime, transition back to the first stimulation regime involves steering anodic current from the second electrode allocation (electrode E2) to the case. Figure 16F shows example waveforms that are formed at electrodes E2, E3, and EC during the transition from the sixth regime back to the first regime. As can be seen, anodic current is gradually steered to the case from electrode E2 to electrode E3 while the full allocation of cathodic current remains constant at electrode E3 throughout the transition.

The sixth stimulation regime sets up a transition back to the first stimulation regime. If a third electrode allocation had been configured via the programming interface 700', the third electrode allocation, rather than the first electrode allocation, would receive all of the cathodic current in the sixth stimulation regime. The anodic current could then be steered from the second electrode allocation to the case to provide cathodic stimulation at the third electrode allocation. Subsequent transitions may mirror those illustrated from the first stimulation regime to the sixth stimulation regime with the exception that the third electrode allocation and another configured electrode allocation (e.g., a fourth configured electrode allocation or the first or second configured electrode allocation) rather than the first and second electrode allocations would be employed to accomplish the same type of locational and polarity weaves shown in the first through sixth stimulation regimes.

As illustrated by the examples, the weave configuration mode enables the user to simply configure two or more electrode allocations while the CP software 96' generates a number of stimulation regimes that weave stimulation in terms of both location and polarity between the configured electrode allocations. Referring back to Figure 14, the weave selector 780 enables the user to then select a particular position that corresponds to a stimulation regime (or a position in transition between two regimes) along the weave path. For example, when the user places the weave selector 780 at the location marked "1," stimulation will be delivered in accordance with the parameters of the first stimulation regime. When the user places the selector 780 at a location between the locations marked "1" and "2," stimulation will be delivered in accordance with the parameters associated with the transition between the first stimulation regime and the second stimulation regime and, more specifically, in accordance with the particular location of the selector between the locations marked as "1" and "2" (i.e., to determine the particular point along the transition). While the selector 780 is illustrated as circular in the depicted embodiment, an alternative selector may be depicted as a line, particularly where there is no defined transition between first and last stimulation regimes. It will be understood that the selector 780 may represent more than six stimulation regimes where more than two electrode allocations are specified.

In addition to selecting a particular position via the selector, the user can additionally opt to select the continuous weave selector 782. Selection of the continuous weave selector 782 will cause stimulation to continuously transition through the defined stimulation regimes. Although not illustrated, in one embodiment, the user may be able to specify a time period over which the transition through the defined stimulation regimes occurs.

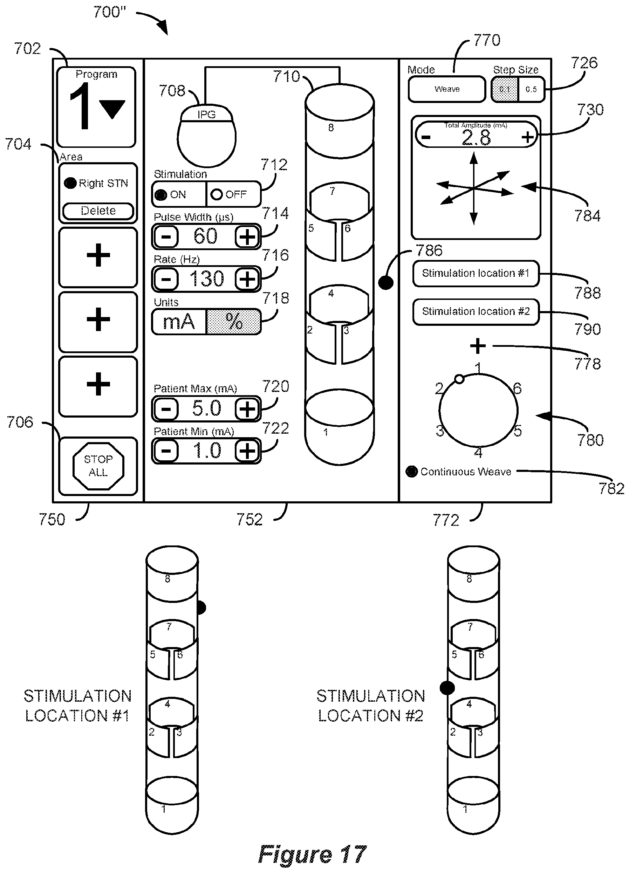

Figure 17 shows a programming interface 700" that includes a modified version of the weave configuration mode. In the modified weave configuration mode, the user selects two or more stimulation locations as opposed to electrode allocations. The electrode steering controls 732, 734, and 736 are therefore replaced by stimulation location adjuster 784. The stimulation location adjuster enables the user to move a stimulation location indicator 786 in three dimensions relative to the lead representation 710. Specifically, the stimulation location adjuster 784 enables the user to translate the stimulation location indicator 786 in three dimensions using Cartesian controls (polar controls could also be used). Just as with the electrode allocations, when the user has moved the stimulation location indicator 786 to the desired location, the stimulation location can be saved by selecting the appropriate stimulation location selector 788 or 790. The bottom portion of Figure 17 shows two example stimulation locations that might be selected by the user.

The allocation of current among the electrodes that most closely approximates a selected stimulation location can be determined using electric field modeling techniques as described in US Patent No. 8,412,345. The same type of techniques may be utilized to determine the electrode allocations for different stimulation locations along a path between the selected stimulation locations such that current can be allocated appropriately between electrodes during transitions between the stimulation locations. Given the calculated electrode allocations that correspond to the selected stimulation locations, the stimulation regimes can be determined in the same manner as described above. Therefore, the modified weave configuration mode enables the user to simply configure two or more stimulation locations while the CP software 96' generates a number of stimulation regimes that weave stimulation in terms of both location and polarity between the configured stimulation locations. The user can then utilize the weave selector 780 and the continuous weave selector 782 in the same manner as described above to customize the therapy that is provided by the IPG. As the stimulation location is moved between selected stimulation locations, it may be desirable to adjust the total stimulation amplitude to accommodate for the recruitment of different neural populations. Amplitude adjustment to maintain stimulation intensity is described in U.S. Patent No. 8,644,947 and it applies equally to anodic stimulation (although different neural models may be necessary for the different stimulation modes due to the different biological mechanisms involved).

As specified above, the interfaces 700' and 700" receive inputs that correspond to a first stimulation location and a second stimulation location (as used herein, stimulation location refers to an input that specifies either an electrode allocation or an actual location of stimulation), defines stimulation regimes corresponding to each of the first stimulation location and the second stimulation location for each of a first stimulation mode and a second stimulation mode (i.e., anodic stimulation and cathodic stimulation at each of the specified locations), defines a path that connects the stimulation regimes (e.g., the path between regimes such as illustrated in Figure 15), receives a selection of a position along the path (e.g., via the selector 780), and communicates stimulation parameters to the IPG 10 based on the selected location along the path.

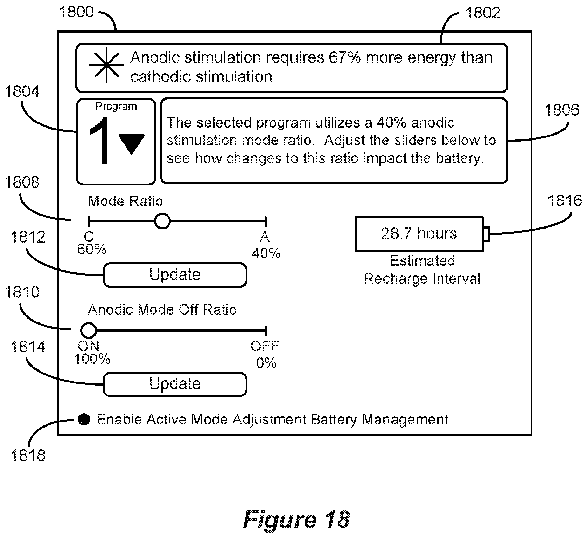

As noted above, due to the different biological mechanisms involved in cathodic and anodic stimulation, it has been observed that anodic stimulation typically requires higher stimulation amplitudes than cathodic stimulation to achieve effective therapy. Thus, anodic stimulation typically requires greater energy than cathodic stimulation. Figure 18 shows an example energy management interface 1800 that enables the user to visualize the manner in which selected stimulation settings impact the IPG 10's battery 14 and to configure battery management settings. The energy management interface 1800 can be utilized when the current type of stimulation is bimodal or of a single mode.

The energy management interface 1800 utilizes energy calculations (or power calculations) to provide information regarding energy usage for the different stimulation modes. In one embodiment, the energy calculations are based on the configured stimulation parameters (stimulation amplitude, frequency, pulse width) for the particular stimulation mode using a predefined estimate of tissue impedance. The power utilized to provide electrical stimulation may be computed using this technique by multiplying the squared stimulation amplitude, the predefined impedance estimate, the pulse width, the frequency, and the mode ratio as will be understood. In another embodiment, the same calculations can be performed but the estimated impedance can be replaced by measured impedance. U.S. Patent No. 9, 061,140 describes a technique for measuring the tissue impedance between electrodes that are used to provide stimulation. In a preferred embodiment, the impedance will be measured for each stimulation mode in each stimulation program as tissue impedance will differ for different stimulation parameters. Moreover, as tissue impedance changes over time, the measured impedance for a particular stimulation mode in a particular stimulation program may comprise an average of a number of impedance measurements taken over time for that particular stimulation mode and stimulation program. In another embodiment, the energy usage for each stimulation mode in each program can be calculated by multiplying the stimulation amplitude for the stimulation mode by the measured compliance voltage that is used when stimulation of that mode is being provided. In such an embodiment, multiple measurements of the compliance voltage for each stimulation mode in each stimulation program may be averaged to determine the particular stimulation mode's compliance voltage as compliance voltage changes over time even for the same set of stimulation parameters.

Regardless of the manner in which energy usage is computed, the energy usage computation can be performed for each configured stimulation mode in each stimulation program. From the calculated values for the different modes in the different stimulation programs, the relative energy usage for the different stimulation modes can be computed. In the illustrated example, this relative energy usage value is displayed within an energy usage indicator 1802. For example, the energy usage indicator 1802 can provide an indication of the relative energy usage for anodic stimulation (across all configured stimulation programs) as compared to cathodic stimulation (across all configured stimulation programs).

The energy management interface 1800 additionally includes a program selector 1804 that enables the mode parameters for a particular program to be displayed. When a particular stimulation program is selected, that program's mode ratio (e.g., the mode ratio specified via the interleave mode ratio selector 832 is displayed in a mode ratio indicator 1806. The energy management interface additionally includes a mode ratio adjuster 1808 and an anodic mode off ratio adjuster 1810 that enable the user to evaluate the effect on battery life for different settings. The mode ratio adjuster 1808 functions in the same manner as the interleave mode ratio selector 832 and updates the mode ratio set by the interleave mode ratio selector 832 for the selected stimulation program. The anodic mode off ratio adjuster 1810 specifies the ratio of time that the mode ratio for the selected stimulation program is set to 100% cathodic stimulation mode. For example, if the anodic mode off ratio adjuster 1810 is set to 100% on, then the mode ratio for the selected stimulation program is not altered. However, if the anodic mode off ratio adjuster 1810 is set to 50%, then the mode ratio for the selected stimulation program is set at the defined ratio value (e.g., 40% anodic and 60% cathodic in the illustrated example) for 50% of the time and is overridden by setting the mode ratio to 0% anodic and 100% cathodic for the other 50% of the time. The anodic mode off ratio adjuster 1810 thus enables a user to specify a proportion of time during which the anodic stimulation mode for a selected stimulation program functions in accordance with the settings prescribed via the bimodal definition interface 836 and a proportion of time during which the anodic stimulation mode for the selected stimulation program is turned off.

In a preferred embodiment, the values selected via the mode ratio adjuster 1808 and the anodic mode off ratio adjuster 1810 are not implemented until the corresponding update selector 1812 or 1814 is selected. Until the settings are accepted via the update selector 1812 or 1814, they are only used to provide a visualization of an impact on energy usage for the proposed modifications. The above-described energy computations are thus updated to determine a revised energy usage value for stimulation given the proposed adjustments that are selected via the mode ratio adjuster 1808 and the anodic mode off ratio adjuster 1810. The revised energy usage value is then utilized in conjunction with other energy usage values to determine the battery life. In the illustrated embodiment, battery life is indicated as an estimated recharge interval via the battery indicator 1816. The estimated recharge interval may specify the estimated time that the IPG 10 might be expected to operate before requiring that its battery 14 be recharged for the current stimulation settings as modified by the proposed adjustments. The estimated recharge interval is obviously relevant only for IPGs with rechargeable batteries and the battery indicator 1816 may instead indicate the estimated remaining battery life if the IPG alternatively includes a primary cell (i.e., non-rechargeable) battery. If the user believes that the proposed adjustments that are entered via the mode ratio adjuster 1808 and/or the anodic mode off ratio adjuster 1810 are justified based on an estimated improvement in battery life, the proposed adjustments may be accepted as described above via the appropriate update selector 1812 or 1814. Upon acceptance, revised stimulation parameters that incorporate the adjustments may be communicated to the IPG 10.

In addition to providing information regarding the energy usage of different stimulation modes and allowing the user to visualize the effects of stimulation changes on battery life, the energy management interface 1800 additionally enables the user to turn on active mode adjustment battery management using the selector 1818. When active mode adjustment battery management is enabled, mode adjustments are automatically instituted by the IPG 10 when the battery level reaches a specified level (e.g., a low level at which it is desirable to adjust the stimulation mode settings to preserve battery life). In one embodiment, the user may be able to specify the battery level (e.g., as a percentage of remaining battery life, early replacement interval, etc.) at which active mode adjustment battery management becomes functional. In one embodiment, when the battery reaches the specified level, the active mode adjustment functionality begins increasing the anodic mode off ratio (i.e., increasing the proportion of time that the mode ratio is overridden to 100% cathodic and 0% anodic) according to a predefined function that increases the anodic mode off ratio as the battery level further declines. As can be seen, the energy management interface 1800 provides the user with significant energy usage information and control over the adjustment of mode settings to achieve a preferable energy usage. Although not specifically illustrated, the energy management interface 1800 can additionally include controls that enable the user to evaluate the effect of mode settings on programming limits (such as current density programming limits) and to adjust mode settings based on the programming limits.

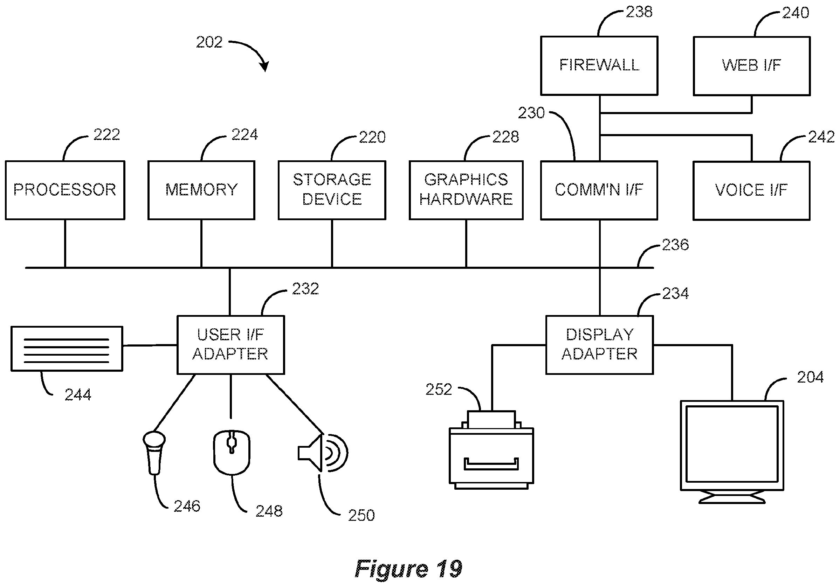

Figure 19 illustrates the various components of an example CP computer 202 that may be configured to execute the improved CP software 96'. The CP computer 202 can include the processor 222, memory 224, storage 220, graphics hardware 228, communication interface 230, user interface adapter 232 and display adapter 234 - all of which may be coupled via system bus or backplane 236. Memory 224 may include one or more different types of media (typically solid-state) used by the processor 222 and graphics hardware 228. For example, memory 224 may include memory cache, read-only memory (ROM), and/or random access memory (RAM). Storage 220 may store media, computer program instructions or software (e.g., CP software 96'), preference information, device profile information, and any other suitable data. Storage 220 may include one or more non-transitory computer-readable storage mediums including, for example, magnetic disks (fixed, floppy, and removable) and tape, optical media such as CD-ROMs and digital versatile disks (DVDs), and semiconductor memory devices such as Electrically Programmable Read-Only Memory (EPROM), Electrically Erasable Programmable Read-Only Memory (EEPROM), and USB or thumb drive. Memory 224 and storage 220 may be used to tangibly retain computer program instructions (e.g., instructions that when executed perform different aspects of the improved CP software 96') or code organized into one or more modules and written in any desired computer programming language. Such computer program instructions are executable by control circuitry 222 to cause the control circuitry to perform various functions defined by the improved CP software 96'. Communication interface 230 (which may comprise, for example, the ports 206 or 208) may be used to connect the CP computer 202 to a network. Communications directed to the CP computer 202 may be passed through a protective firewall 238. Such communications may be interpreted via web interface 240 or voice communications interface 242. Illustrative networks include, but are not limited to: a local network such as a USB network; a business' local area network; or a wide area network such as the Internet. User interface adapter 232 may be used to connect a keyboard 244, microphone 246, pointer device 248, speaker 250 and other user interface devices such as a touch-pad and/or a touch screen (not shown). Display adapter 234 may be used to connect display 204 and printer 252.

Processor 222 may include any programmable control device. Processor 222 may also be implemented as a custom designed circuit that may be embodied in hardware devices such as application specific integrated circuits (ASICs) and field programmable gate arrays (FPGAs). The CP computer 202 may have resident thereon any desired operating system.

While the CP system 200 has been described and illustrated as communicating directly with the IPG 10, the CP system 200 may additionally or alternatively be configured to communicate with different types of neurostimulators. For example, the CP system 200 may interface with an external trial stimulator that mimics the operation of the IPG 10 but that is positioned outside of the body to evaluate therapies during a trial phase. Moreover, while the system has been described in terms of its execution on the CP computer, the improved software 96', or portions thereof, may also be executed on a different device such as the external controller 40. As will be understood, the improved software 96' may be stored on a medium such as a CD or a USB drive, pre-loaded on a computing device such as the CP computer 202, or made available for download from a program repository via a network connection The invention is defined by claim 1. Preferred embodiments are defined in the dependent claims.

## Claims

1. A system comprising:
an implantable medical device that is connectable to an electrode lead having a plurality of electrodes; and
a non-transitory computer-readable medium having instructions that are executable by control circuitry to cause the control circuitry to:
generate a graphical user interface that is configured to receive one or more inputs to specify one or more parameters of a gradual transition over a duration of the transition between a first stimulation mode and a second stimulation mode, wherein the first stimulation mode defines stimulation of a first polarity to be issued at a set of the electrodes and the second stimulation mode defines stimulation of a second polarity to be issued at the set of the electrodes, wherein the first polarity is opposite of the second polarity; and
communicate stimulation parameters based on the one or more parameters of the transition to the implantable medical device.

2. The system of claim 1, wherein the one or more inputs comprise an input that specifies the duration of the transition.

3. The system of claim 1 or claim 2, wherein the one or more inputs comprise an input that specifies a type of the transition.

4. The system of claim 3, wherein the type is selectable as either a linear transition type or a user-customizable transition type.

5. The system of claim 4, wherein the linear transition type specifies a linear increase of an adjustment variable of the first stimulation mode and a linear decrease of the adjustment variable of the second stimulation mode over the duration of the transition.

6. The system of claim 5, wherein the one or more inputs comprise an input that specifies the adjustment variable.

7. The system of claim 6, wherein the adjustment variable is selectable as either pulse width or pulse amplitude.

8. The system of claim 4, wherein the user-customizable transition type specifies a user-customizable adjustment of an adjustment variable of the first stimulation mode and the second stimulation mode over the duration of the transition.

9. The system of claim 8, wherein the graphical user interface is configured to receive one or more sets of values that specify a proportion of a configured value of the adjustment variable over the duration of the transition.

10. The system of claim 9, wherein a first one of the sets of values specifies the proportion as increasing from zero to unity and a second one of the sets of values specifies the proportion as decreasing from unity to zero.

11. The system of claim 9, wherein a first one of the sets of values specifies the proportion of the configured value of the adjustment variable for the first stimulation mode and a second one of the sets of values specifies the proportion of the configured value of the adjustment variable for the second stimulation mode.

12. The system of claim 9, wherein a first one of the sets of values specifies the proportion of the configured value of the adjustment variable for a currently-active one of the first stimulation mode and the second stimulation mode and a second one of the sets of values specifies the proportion of the configured value of the adjustment variable for a currently-inactive one of the first stimulation mode and the second stimulation mode.

13. The system of claim 8, wherein the one or more inputs comprise an input that specifies the adjustment variable.

14. The system of claim 13, wherein the adjustment variable is selectable as either pulse width or pulse amplitude.

15. The system of any of claims 1 to 14, wherein the one or more inputs comprise an input that specifies a time apportionment of the first stimulation mode and the second stimulation mode during the duration of the transition.

## Patentansprüche

1. System aufweisend:
eine implantierbare medizinische Vorrichtung, die an einen Elektrodenleiter anschließbar ist, der mehrere Elektroden hat; und
ein nichtflüchtiges computerlesbares Medium mit Befehlen, die von einer Steuerschaltung ausführbar sind, um die Steuerschaltung zu veranlassen:
eine graphische Benutzerschnittstelle zu erzeugen, die konfiguriert ist, eine oder mehrere Eingaben zu empfangen, um einen oder mehrere Parameter eines allmählichen Übergangs über eine Dauer des Übergangs zwischen einem ersten Stimulationsmodus und einem zweiten Stimulationsmodus zu spezifizieren, wobei der erste Stimulationsmodus eine an einem Satz der Elektroden auszugebende Stimulation einer ersten Polarität definiert und der zweite Stimulationsmodus eine an dem Satz der Elektroden auszugebende Stimulation einer zweiten Polarität definiert, wobei die erste Polarität entgegengesetzt zur zweiten Polarität ist; und
Stimulationsparameter, die auf dem einen oder den mehreren Parametern des Übergangs basieren, zu der implantierbaren medizinischen Vorrichtung zu kommunizieren.

2. System nach Anspruch 1, wobei die eine oder die mehreren Eingaben eine Eingabe aufweisen, die die Dauer des Übergangs spezifiziert.

3. System nach Anspruch 1 oder Anspruch 2, wobei die eine oder die mehreren Eingaben eine Eingabe aufweisen, die einen Typ des Übergangs spezifiziert.

4. System nach Anspruch 3, wobei der Typ als entweder ein linearer Übergangstyp oder ein benutzer-individualisierbarer Übergangstyp auswählbar ist.

5. System nach Anspruch 4, wobei der lineare Übergangstyp eine lineare Zunahme einer Anpassungsvariablen des ersten Stimulationsmodus und eine lineare Abnahme der Anpassungsvariablen des zweiten Stimulationsmodus über die Dauer des Übergangs spezifiziert.

6. System nach Anspruch 5, wobei die eine oder die mehreren Eingaben eine Eingabe aufweisen, die die Anpassungsvariable spezifiziert.

7. System nach Anspruch 6, wobei die Anpassungsvariable als entweder Pulsbreite oder Pulsamplitude auswählbar ist.

8. System nach Anspruch 4, wobei der benutzer-individualisierbare Übergangstyp eine benutzer-individualisierbare Anpassung einer Anpassungsvariablen des ersten Stimulationsmodus und des zweiten Stimulationsmodus über die Dauer des Übergangs spezifiziert.

9. System nach Anspruch 8, wobei die graphische Benutzerschnittstelle konfiguriert ist, einen oder mehrere Wertesätze zu empfangen, die einen Anteil eines konfigurierten Werts der Anpassungsvariablen über die Dauer des Übergangs spezifizieren.

10. System nach Anspruch 9, wobei ein erster der Wertesätze denjenigen Anteil, der von Null auf Eins zunimmt, spezifiziert und ein zweiter der Wertesätze denjenigen Anteil, der von Eins auf Null abnimmt, spezifiziert.

11. System nach Anspruch 9, wobei ein erster der Wertesätze den Anteil des konfigurierten Werts der Anpassungsvariablen für den ersten Stimulationsmodus spezifiziert und ein zweiter der Wertesätze den Anteil des konfigurierten Werts der Anpassungsvariablen für den zweiten Stimulationsmodus spezifiziert.

12. System nach Anspruch 9, wobei ein erster der Wertesätze den Anteil des konfigurierten Werts der Anpassungsvariablen für einen aktuell aktiven des ersten Stimulationsmodus und des zweiten Stimulationsmodus spezifiziert und ein zweiter der Wertesätze den Anteil des konfigurierten Werts der Anpassungsvariablen für einen aktuell inaktiven des ersten Stimulationsmodus und des zweiten Stimulationsmodus spezifiziert.

13. System nach Anspruch 8, wobei die eine oder die mehreren Eingaben eine Eingabe aufweisen, die die Anpassungsvariable spezifiziert.

14. System nach Anspruch 13, wobei die Anpassungsvariable als entweder eine Pulsbreite oder eine Pulsamplitude auswählbar ist.

15. System nach einem der Ansprüche 1 bis 14, wobei die eine oder die mehreren Eingaben eine Eingabe aufweisen, die eine Zeitaufteilung des ersten Stimulationsmodus und des zweiten Stimulationsmodus während der Dauer des Übergangs spezifiziert.

## Revendications

1. Système comprenant :
un dispositif médical implantable qui peut être connecté à un fil d'électrode ayant une pluralité d'électrodes ; et
un support non transitoire lisible par ordinateur ayant des instructions qui sont exécutables par un circuit de commande pour amener le circuit de commande à :
générer une interface utilisateur graphique qui est configurée pour recevoir une ou plusieurs entrées pour spécifier un ou plusieurs paramètres d'une transition graduelle sur une durée de la transition entre un premier mode de stimulation et un deuxième mode de stimulation, dans lequel le premier mode de stimulation définit une stimulation d'une première polarité à délivrer au niveau d'un ensemble d'électrodes et le deuxième mode de stimulation définit une stimulation d'une deuxième polarité à délivrer au niveau de l'ensemble d'électrodes, dans lequel la première polarité est opposée à la deuxième polarité ; et
communiquer au dispositif médical implantable des paramètres de stimulation sur la base des un ou plusieurs paramètres de la transition.

2. Système selon la revendication 1, dans lequel les une ou plusieurs entrées comprennent une entrée qui spécifie la durée de la transition.

3. Système selon la revendication 1 ou la revendication 2, dans lequel les une ou plusieurs entrées comprennent une entrée qui spécifie un type de la transition.

4. Système selon la revendication 3, dans lequel le type peut être sélectionné comme étant soit un type de transition linéaire ou un type de transition personnalisable par l'utilisateur.

5. Système selon la revendication 4, dans lequel le type de transition linéaire spécifie une augmentation linéaire d'une variable de réglage du premier mode de stimulation et une diminution linéaire de la variable de réglage du deuxième mode de stimulation sur la durée de la transition.

6. Système selon la revendication 5, dans lequel les une ou plusieurs entrées comprennent une entrée qui spécifie la variable de réglage.

7. Système selon la revendication 6, dans lequel la variable de réglage peut être sélectionnée comme étant soit une largeur d'impulsion, soit une amplitude d'impulsion.

8. Système selon la revendication 4, dans lequel le type de transition personnalisable par l'utilisateur spécifie un réglage personnalisable par l'utilisateur d'une variable de réglage du premier mode de stimulation et du deuxième mode de stimulation sur la durée de la transition.

9. Système selon la revendication 8, dans lequel l'interface utilisateur graphique est configurée pour recevoir un ou plusieurs ensembles de valeurs qui spécifient une proportion d'une valeur configurée de la variable de réglage sur la durée de la transition.

10. Système selon la revendication 9, dans lequel un premier des ensembles de valeurs spécifie la proportion comme augmentant de zéro à l'unité et un deuxième des ensembles de valeurs spécifie la proportion comme diminuant de l'unité à zéro.

11. Système selon la revendication 9, dans lequel un premier des ensembles de valeurs spécifie la proportion de la valeur configurée de la variable de réglage pour le premier mode de stimulation et un deuxième des ensembles de valeurs spécifie la proportion de la valeur configurée de la variable de réglage pour le deuxième mode de stimulation.

12. Système selon la revendication 9, dans lequel un premier des ensembles de valeurs spécifie la proportion de la valeur configurée de la variable de réglage pour un mode actuellement actif parmi le premier mode de stimulation et le deuxième mode de stimulation et un deuxième des ensembles de valeurs spécifie la proportion de la valeur configurée de la variable de réglage pour un mode actuellement inactif parmi le premier mode de stimulation et le deuxième mode de stimulation.

13. Système selon la revendication 8, dans lequel les une ou plusieurs entrées comprennent une entrée qui spécifie la variable de réglage.

14. Système selon la revendication 13, dans lequel la variable de réglage peut être sélectionnée comme étant soit une largeur d'impulsion, soit une amplitude d'impulsion.

15. Système selon l'une quelconque des revendications 1 à 14, dans lequel les une ou plusieurs entrées comprennent une entrée qui spécifie une répartition temporelle du premier mode de stimulation et du deuxième mode de stimulation pendant la durée de la transition.
